(19) Europäisches Patentamt
European Patent Office
Office européen des brevets

(11) Veröffentlichungsnummer: **0 219 034 B1**

# (12) EUROPÄISCHE PATENTSCHRIFT

(45) Veröffentlichungstag der Patentschrift: **24.07.91**

(21) Anmeldenummer: **86113833.7**

(22) Anmeldetag: **06.10.86**

(51) Int. Cl.5: **C12N 15/55**, C12N 9/86, C12N 1/20, C12P 13/04, //(C12N1/20,C12R1:19)

## (54) Verfahren zur Herstellung von mesophilen Mikroorganismen, die eine bei höherer Temperatur aktive D-Hydantoinase enthalten.

(30) Priorität: **09.10.85 DE 3535987**

(43) Veröffentlichungstag der Anmeldung:
**22.04.87 Patentblatt 87/17**

(45) Bekanntmachung des Hinweises auf die Patenterteilung:
**24.07.91 Patentblatt 91/30**

(84) Benannte Vertragsstaaten:
**AT BE CH DE FR GB IT LI NL**

(56) Entgegenhaltungen:
**DE-A- 3 031 151**

**PATENT ABSTRACTS OF JAPAN, Band 10, Nr. 112 (C-3429[2169], 25. April 1986; & JP - A - 60 241 888 (DENKI KAGAKU KOGYO K.K.) 30.11.1985**

**PATENT ABSTRACTS OF JAPAN, Band 4, Nr. 159 (C-30)[641], 6. November 1980; & JP - A - 55 104 890 (KENEGAFUCHI KAGAKU KOGYO K.K.) 11.08.1980**

(73) Patentinhaber: **BASF Aktiengesellschaft**
**Carl-Bosch-Strasse 38**
**W-6700 Ludwigshafen(DE)**

(72) Erfinder: **Jacob, Elard, Dr.**
**Haendelstrasse 1**
**W-6719 Eisenberg(DE)**
Erfinder: **Henco, Karsten, Dr.**
**Schlikumer Weg 23**
**W-4006 Erkrath(DE)**
Erfinder: **Marcinowski, Stefan, Dr.**
**Weimarer Strasse 82**
**W-6700 Ludwigshafen(DE)**
Erfinder: **Schenk, Gerhard, Dr.**
**Verschaffeltstrasse**
**W-6830 Schwetzingen(DE)**

EP 0 219 034 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

PATENT ABSTRACTS OF JAPAN, Band 3, Nr. 104 (C-057) 4. September 1979; & JP - A - 54 084 086 (KANEGAFUCHI CHEM IND CO LTD) 04.07.1979

PATENT ABSTRACTS OF JAPAN, Band 3, Nr.11 (C-035) 30. Januar 1979; & JP - A - 53 136 583 (KANEGAFUCHI CHEM IND CO LTD) 29.11.1978

## Beschreibung

Dihydropyrimidinasen [EC 3.5.2.2] katalysieren die Spaltung razemischer Hydantoine in die entsprechenden D-N- bzw. L-N-Carbamoyl-alpha-aminosäuren. D-N-Carbamoyl-alpha-aminosäuren sind wichtige Zwischenprodukte zur Herstellung von D-Aminosäuren, die als wertvolle Ausgangsstoffe für die Produktion halbsynthetischer Penicilline und Cephalosporine dienen.

Eine Dihydropyrimidinase aus einem thermophilen Mikroorganismus wurde beschrieben (I), die in der Lage ist, D,L-Hydantoine stereoselektiv in D-N-Carbamoyl-alpha-aminosäuren zu spalten, wobei - bedingt durch die hohe Umwandlungstemperatur, bei der das Enzym aktiv ist (40 bis 90 °C) - das nicht gespaltene L-Hydantoin spontan nachrazemisiert. Diese Dihydropyrimidinase, die auf Grund ihrer Spezifität für Hydantoine auch als D-Hydantoinase zu bezeichnen ist, läßt sich durch Fermentation von asporogenen, gramnegativen thermophilen Mikroorganismen oder thermophilen Bacillacaeen gewinnen und als Detergensrohlysat, ggf. nach Immobilisierung, für die Spaltung von D,L-Hydantoinen einsetzen. Die verwendeten thermophilen Mikroorganismen wachsen jedoch nur zu einer geringen Zelldichte. Weiterhin beträgt der Gehalt an D-Hydantoinase nur einige Promille des Gesamtproteins der Mikroorganismen.

Eine Erhöhung der Fermentationsausbeute ist prinzipiell auf zwei Wegen möglich:

1. Man kann versuchen, durch Mutagenese nach chemischen oder physikalischen Methoden die Mikroorganismen so zu verändern, daß sie eine bedeutend höhere Ausbeute an Enzym liefern. Die Suche nach wesentlich verbesserten Mikroorganismen ist jedoch sehr aufwendig und in ihrem Ergebnis ungewiß.
2. Man kann versuchen, das für die Enzymbildung verantwortliche Gen aus einem Mikroorganismus zu isolieren und so zu verändern, daß es nach Implantation in den ursprünglichen Spenderorganismus oder einen anderen Mikroorganismus, der dieses Gen aufnehmen, stabil weitervererben und funktionsfähig erhalten kann, zu einer höheren Enzymbildung führt.

Es können auch gezielte Veränderungen im Bereich der mit dem Gen assoziierten DNA-Sequenzen oder im Genbereich selber vorgenommen werden, um die Aktivität der Enzymbildung zu erhöhen und das Enzym zu stabilisieren.

Schließlich kann die Enzymbildung der Zellen auch dadurch gesteigert werden, daß man in jeder Zelle die Zahl identischer Gene erhöht, die für die Enzymbildung verantwortlich sind.

Obwohl bereits eine Reihe von Genverpflanzungen mit Erfolg durchgeführt worden sind (II-VI), ist nicht vorhersehbar, ob in bestimmten Fällen eine Genverpflanzung zu dem gewünschten Erfolg führt.

Gegenstand der Erfindung ist ein Verfahren zur Herstellung von mesophilen Mikroorganismen, die eine bei höherer Temperatur aktive D-Hydantoinase enthalten, welches darin besteht, daß man

a) die DNA aus einem D-Hydantoin spaltenden thermophilen Mikroorganismus isoliert und in Bruchstücke zerlegt,

b) die so erhaltenen DNA-Bruchstücke mit einem Klonierungsvektor kombiniert,

c) die so erhaltenen rekombinanten Klonierungsvektoren in einen mesophilen Mikroorganismus integriert und

d) solche Mikroorganismen selektiert, die enzymatisch aktive D-Hydantoinase exprimieren.

Die Erfindung betrifft weiter aus thermophilen Mikroorganismen erhältliche D-Hydantoinase kodierende DNA-Sequenzen und Klonierungsvektoren, die diese Sequenzen enthalten, sowie diese Klonierungsvektoren enthaltende mesophile Mikroorganismen und deren Verwendung zur Herstellung von thermophilen D-Hydantoinasen bzw. zur Spaltung von D,L-Hydantoinen.

Zur Herstellung der durch Genmanipulation erhältlichen D-Hydantoine spaltenden mesophilen Mikroorganismen geht man von thermophilen Mikroorganismen aus, die D-Hydantoine spalten. Solche Mikroorganismen sind beispielsweise Thermus spec. und die thermophilen Vertreter der Familie der Bacillacaeen. Sie lassen sich durch kurzzeitiges Erhitzen erdehaltiger oder wäßriger Proben aus nährstoffreichen, wäßrigen Biotopen auf etwa 100 °C, Inkubation in Nährmedium bei etwa 60 °C und Selektion auf D-Hydantoinaseaktivität gewinnen (I). Zwei solcher Stämme sind hinterlegt (CBS 303.80 und CBS 363.80).

Aus diesen Mikroorganismen wird die DNA, die sogenannte "Spender-DNA", isoliert, indem man deren Zellwände beispielsweise mit einem Enzym wie Lysozym und/oder mit einem Detergens zerstört, aus dem so erhaltenen Lysat die Hauptmenge Protein mit phenolhaltigen Lösungen ausfällt und anschließend restliches Protein durch enzymatische Spaltung und anschließende Dialyse entfernt. Aus der so erhaltenen Lösung kann die Spender-DNA durch Gleichgewichtsgradientenzentrifugtion und anschließende Dialyse rein erhalten werden.

Zur Isolierung bzw. Anreicherung des D-Hydantoinasegens muß die Spender-DNA fragmentiert werden. Das kann physikalisch durch Einwirken von Scherkräften oder chemisch durch enzymatische Spaltung erfolgen.

Die physikalische Fragmentierung erfolgt beispielsweise durch Ultraschallbehandlung, hochtouriges Rühren in einem Homogenisator, Pressen durch englumige Kanülen oder wiederholtes Einfrieren und Auftauen der DNA (VII).

Die enzymatische Fragmentierung der Spender-DNA kann beispielsweise mit statistisch spaltenden DNAasen vorgenommen werden unter Bedingungen, bei denen vorzugsweise Doppelstrangbrüche auftreten (VIII).

Anschließend muß die fragmentierte Spender-DNA durch Anfügen von "Linker"- oder "Adaptor"-Sequenzen in eine Form gebracht werden, in der sie als integraler Bestandteil in eine Vektor-DNA aufgenommen werden kann. Spender- und Vektor-DNA können auch miteinander verbunden werden, indem man beide mit zueinander komplementären Basen verlängert und sie aneinanderkoppelt.

Besonders bewährt hat sich die Spaltung der Spender-DNA mit einer Restriktionsendonuklease, die an den Enden der DNA-Bruchstücke bestimmte, nur von der Art der Restriktionsendonuklease abhängige Nukleotidsequenzen freilegt. Bei der Verwendung der Plasmide pBR322 oder pBR327 (IX-X) oder davon abgeleiteter Plasmide wie pHC79 (XI) als Vektor-DNA kommen als Restriktionsendonukleasen insbesondere EndoR.BamHI, EndoR.HindIII, EndoR.EcoRI oder EndoR.PstI in Frage. [Die Bezeichnung der Enzyme wird meistens durch weglassen der Typenbezeichnung (EndoR) abgekürzt.] Diese schneiden die genannten Plasmide nur einmal und führen zu einer linearen Vektor-DNA, in die sich die Spender-DNA-Bruchstücke besonders gut steuerbar integrieren lassen. Auch Kombinationen von bestimmten Restriktionsendonukleasen, die homologe Endsequenzen der gespaltenen DNA freisetzen, kommen in Frage; z.B. kann die Spender-DNA mit der an vielen Erkennungsstellen schneidenden EndoR.Sau3A fragmentiert werden. Anschließend lassen sich die Bruchstücke in mit EndoR.BamHI geschnittene Vektor-DNA durch Einwirkung von DNA-Ligase [EC 6.5.1.1] integrieren. Führt man die Fragmentierung der Spender-DNA mit einer niedrigen Konzentration einer solchen, an vielen Stellen schneidenden Restriktionsendonuklease durch, lassen sich quasistatistisch erzeugte DNA-Fragmente gewinnen, wobei die Größe der Fragmente von der Enzymkonzentration und seiner Einwirkungsdauer abhängt. Auf diese Weise lassen sich auch DNA-Fragmente klonieren, die auf Grund ihrer spezifischen Basensequenz für die seltener spaltenden Restriktionsendonukleasen wie die oben aufgeführten keine oder nicht in ausreichender Nähe zu dem gesuchten Gen liegende Erkennungsstellen aufweisen.

Die Bruchstücke der Spender-DNA werden in die linearisierte Vektor-DNA eingebaut. Das kann mit DNA-Ligasen geschehen, die aus gegebenenfalls mit T4 infizierten E.coli-Zellen gewonnen werden können. Für den Einbau wird ein Kofaktor ($NAD^+$ bzw. ATP) benötigt.

Als Vektor-DNA eignet sich DNA aus Plasmiden, Cosmiden oder Bakteriophagen. Es empfiehlt sich, für die ersten Einbauschritte linearisierte Cosmide oder die DNA des Bakteriophagen λ zu verwenden und für die späteren Einbauschritte Plasmide, weil Cosmide oder Bakterophagen λ DNA sehr viel größere DNA-Fragmente zu klonieren erlauben als Plasmid-DNA. Typische Maximalgrößen für DNA-Fragmente, die in einem Cosmid- oder Bakteriophagen-Vaktor kloniert werden können, liegen zwischen etwa 50-23 kb (1 kb entspricht 1000 Basenpaaren doppelsträngiger DNA). Damit wird die Zahl unabhängiger Klone, die auf Expression von D-Hydantoinase geprüft werden müssen, niedrig gehalten. Beispielsweise ist bei einer integrierten Fragmentgröße von 40 kb in einem Cosmidvektor die gesamte DNA eines Mikroorganismus in etwa 350 unabhängigen Klonen mit einer Wahrscheinlichkeit von 99 % erhalten, während bei einer Fragmentgröße von 6 kb, die im typischen Größenbereich für eine Plasmidklonierung liegt, die Zahl der zu untersuchenden Klone auf etwa 2.300 ansteigt.

Jedoch müssen nach Auffinden der gesuchten kodierenden DNA-Sequenz die benachbarten unerwünschten DNA-Sequenzen, die zunächst zwangsläufig mitkloniert wurden, entfernt werden, um eine möglichst hohe Stabilität zu erzielen. Hierfür eignet sich der Einbau in Plasmide am besten, zumal auch die häufig notwendige Manipulation der DNA in Form von Restriktionsenzym-Verdau und Ligation durch die reduzierte Größe der DNA erleichtert wird.

Der so erhaltene Hybrid-Vektor wird in eine geeignete Wirtszelle gebracht. Solche sind E.coli-Zellen mit definierten genetischen Marken, insbesondere solche, die einen Defekt im Restriktions/Modifikationssystem aufweisen ($r^-m^-$), rekombinationsdefizient sind ($recA^-$) und gegebenenfalls einen hitzelabilen Lambda-Repressor ($c_I857$) aufweisen.

Besonders geeignete Wirtszellen sind vorzugsweise E.coli HB 101, NF1, W6(λrex) und N4830.

Der Nachweis der gelungenen Genklonierung hängt maßgeblich in dieser Stufe davon ab, ob ein spezifisches Nachweisreagens für das Gen zur Verfügung steht. Im allgemeinen kann dies eine Nukleinsäure oder ein Nukleinsäurefragment sein, das aus der Spenderzelle isoliert wird oder ein bereits früher kloniertes Teilstück des betreffenden Gens oder ihm benachbarter Sequenz darstellt oder das chemisch anhand einer partiellen Aminosäuresequenz des Proteinproduktes synthetisiert wurde, für das das entsprechende Gen kodiert. Schließlich kann auch die Kapazität des Gens genutzt werden, in der artfremden Empfängerzelle das von ihm spezifizierte Proteinprodukt zu produzieren, wobei in diesem Fall ein Antikörper zum Nachweis des gebildeten Proteins zur Verfügung stehen muß oder das gebildete Protein gegebenenfalls über seine enzymatische Aktivität nachgewiesen werden kann. In beiden Fällen muß das gesuchte Gen (A) Signalsequenzen mitbringen, die in der Empfängerzelle als Transkriptionspromotor, -terminator bzw. Ribosomenbindungsstelle funktionieren können (zur Definition dieser Begriffe vgl. XII). Aus diesem Aspekt wie auch bedingt durch den Screeningaufwand über einen enzymatischen Test ist es vorteilhaft, die anfänglichen Klonierungen mit großen DNA-Fragmenten durchzuführen, für die sich eine Klonierung in einem Cosmid- oder Phagenvektor anbietet.

Um die Expression des erhaltenen Gens (A) weiter zu verbessern, können die genannten Signalsequenzen auch durch ein anderes Gen (B) zur Verfügung gestellt werden. Solche andere Gene (B) sind beispielsweise das DNA-Äquivalent eines Teilstückes der MS2-Replikase (XXVI) und das cro-Genfragment des Bakteriophagen λ (XXXVI).

Zunächst ist es jedoch zweckmäßig, die Gene aus dem Cosmid in ein kleines Plasmid einzuführen. Hierzu wird die Cosmid-DNA, die unter anderem das Gen (A) enthält, isoliert und in mehreren Ansätzen unterschiedlich lange mit einer DNAase geschnitten. Die Ansätze werden vereinigt und elektrophoretisch oder chromatographisch nach der Größe fraktioniert. Die DNA-Fraktion des Molekulargewichtsbereichs, der der Größe des intakten Gens entspricht, wird isoliert und gereinigt. Die so gewonnene DNA wird mit linearisierter, mindestens einfach resistenzmarkierter Plasmid-DNA ligiert. Der Ligationsansatz wird in Bakterienzellen transformiert. Die so erhaltenen Zellen werden in einem Medium kloniert, welches den Stoff enthält, gegen das das verwendete Plasmid der Wirtszelle Resistenz vermittelt. Die Klone, die Hydantoinase bilden, werden isoliert.

Ein solcher Klon wird zur weiteren Expressionsoptimierung verwendet. Dazu wird zunächst das darin enthaltene Plasmid in bekannter Weise isoliert und linearisiert. Die so erhaltene lineare DNA wird mit einer von den Enden des Moleküls progressiv einwirkenden DNAase verdaut und schließlich nach Anfügen von DNA-Linkern wieder zyklisiert. Die Linearisierung zu Anfang dieses Verfahrens wird mit solchen Restriktionsenzymen vorgenommen, daß eine Eliminierung unerwünschter DNA-Sequenzen vor und nach dem Gen durch die progressiv wirkende DNAase im zweiten Schritt erfolgt.

Die durch die Ligation rezirkularisierten Plasmide werden anschließend in ein Bakterium transferiert und kloniert. Die D-Hydantoinase exprimierenden Klone werden so selektiert, daß sie möglichst wenig ursprüngliche DNA außerhalb der D-Hydantoinase-Kodiersequenz enthalten. Hierzu empfiehlt es sich, einen Klon, der möglichst wenig ursprüngliche DNA zwischen dem 5′-Ende und einer Enzymspaltstelle enthält, mit einem solchen zu kombinieren, der möglichst wenig ursprüngliche DNA zwischen dem 3′-Ende und einer Enzymspaltstelle enthält. Das Kombinationsprodukt wird in üblicher Weise vermehrt, isoliert und das D-Hydantoinase-Gen enzymatisch herausgeschnitten. Dies wird in ein geeignetes Plasmid integriert, das einen starken Promotor und eine Ribosomenbindungsstelle hoher Aktivität aufweist, z.B. die pPCLC24-Derivate (XXVI) pEX31 oder das Plasmid pCL 547 (XXXVI).

Die so erhaltenen Plasmide werden anschließend in ein Bakterium transferiert. Da die Promotoren der genannten Plasmide durch den Lambda-Repressor $c_I$ reguliert werden, sollten die für die Transformation verwendeten E.coli-Stäme ein mutiertes $c_I$-Gen besitzen, das zu einem temperaturlabilen Repressor führt. In solchen Zellen können die Promotoren durch erhöhte Temperatur induziert werden. Die plasmidhaltigen Bakterien werden schließlich nach D-Hydantoinase-Enzymaktivität selektiert.

Die Klonierung der Gene wurde über die gebildeten D-Hydantoinaseproteine mittels spezifischer polyklonaler Antikörper sowie über ihre enzymatische Aktivität nachgewiesen.

Überraschenderweise exprimieren sich die D-Hydantoinasegene, die aus thermophilen Mikroorganismen isoliert wurden, in den mesophilen E.coli Empfängerzellen, ohne daß dafür fremde Singalsequenzen zur Verfügung gestellt werden müssen. Allerdings erwies sich die klonierte DNA-Sequenz des Stammes CBS 303.80 in verschiedenen Stadien der Konstruktion nach der Teildeletion der mitklonierten 3′genproximalen DNA-Sequenzen als instabil und mußte durch eingehende Restriktions- und Sequenzanalyse aus den entstehenden Mutanten selektiert werden. Bei einer Restlänge der 3′-Sequenzen von 25 bis 125 Nukeotiden nach dem Translationsstopsignal des D-Hydantoinasegens von CBS 303.80 und ca. 250 Nukleotiden vor dem Startsignal (5′-genproximal) war das Gen jedoch wieder stabil. Obwohl schließlich bei der Expressionsoptimierung für die beiden D-Hydantoinase Gene eine Koppelung an ein anderes Gen (MS2-Replicasefragment in den pEX-Konstruktionen, cro-Genfragment in den pCL-Konstruktionen (vgl. Beispiel 1.8 und Beispiel 2.6) angestrebt wurde, wurde nur ein Fall gefunden, bei dem tatsächlich ein Fusionsprotein nachweisbar war. Dieses Fusionsprotein hatte ebenfalls D-Hydantoinaseaktivität. In allen anderen Fällen wurden D-Hydantoinasen nativer Länge gefunden. Überraschenderweise war die Synthese der Enzyme in diesen Konstruktionen (temperatur)induzierbar. Sie führte zu einer 4- bis 40fachen höheren Enzymaktivität (bezogen auf Zellmasse) als in den Ausgangsstämmen (40fach höher für CBS 303.80).

In den im folgenden angeführten Beispielen wird die erfolgreiche Klonierung von zwei nicht miteinander verwandten D-Hydantoinase Genen beschrieben, die nur in den ersten 19 Aminosäuren des Aminoterminus eine begrenzte Homologie (52 %) aufweisen (Fig. 1; die Positionen, an denen identische Aminosäuren stehen, sind eingerahmt).

Beispiel 1

1.1 Isolierung der DNA

200 ml Castenholz-Nährmedium (XIII) (= 10 ml Basalmedium [889 ml $H_2O$, 33,4 mg $FeCl_3 \times 6H_2O$, 20,48 mg $MgSO_4 \times 7H_2O$, 0,8 g NaCl, 10,3 g $KNO_3$, 68,9 g $NaNO_3$, 10 g Titriplex III, 7,59 g $CaSO_4 \times 2H_2O$, 100 ml Spurenelementlösung [enthaltend pro Liter 0,5 ml $H_2SO_4$ konz., 2,69 g $MnCl_2 \times 4H_2O$, 0,72 g $ZnSO_4 \times 7H_2O$, 0,5 g $H_3BO_3$, 78 mg CuCl, 28,75 mg $Na_2MoO_4 \times 2H_2O$, 94,94 mg $CoSO_4 \times 7H_2O$], 2,5 g Hefeextrakt, 2,5 g Bactotrypton, 0,258 g $Na_3PO_4 \times 12H_2O$pro l) wurden mit 10 ml Zellen des asporogenen gramnegativen thermophilen Bakterienstammes CBS 303.80 aus einer frischen Übernachtkultur beimpft und 24 h bei 60 °C geschüttelt. Die Bakterienkultur wurde 20 min bei 4000 bis 8000 g abzentrifugiert, das Sediment in 200 ml Lösung A (50 mM Tris-HCl, pH 8, 10 % Saccharose) resuspendiert, erneut abzentrifugiert und in 16 ml Lösung A resuspendiert. Unter Mischen wurden 3,2 ml einer Hühnereiweiß-Lysozym-Lösung (5 mg/ml in Lösung A) zugefügt. Nach 15 min Inkubation bei Raumtemperatur wurden 4 ml 0,5 M EDTA-Lösung, pH 8,0, zugegeben, die Lösung durch Schütteln gemischt und nach weiteren 5 min bei Raumtemperatur 20 ml Lösung B (50 mM Tris-HCl, pH 8, 10 mM EDTA, 0,5 % Triton X100) unter rascher Vermischung zupipettiert. Unmittelbar danach wurden 2,6 ml 10 %ige Natriumdodecylsulfat-Lösung zugefügt. Nach Mischen wurde der zähviskose Zellaufschluß 10 min bei 60 °C gehalten. Nach Zugabe von 5 ml 5 M Natriumperchlorat-Lösung wurde das Zellysat mit 50 ml Phenol/Chloroform/Isoamylalkohol [25 Volumenteile Phenol (mit 0,1 M Tris-HCl, pH 7,4, 0,05 M NaCl, 10 mM EDTA und 0,01 % $\beta$-Hydroxychinolin äquilibriert)], 24 Volumenteile Chloroform, 1 Volumenteil Isoamylalkohol] bei 40 °C 30 min geschüttelt. Die Emulsion wurde durch 10 min Zentrifugation bei 4000 g getrennt. Die isolierte Oberphase wurde zweimal wie oben beschrieben mit Phenol/Chloroform/Isoamylalkohol und anschließend mit demselben Volumen Chloroform extrahiert. Die wäßrige Oberphase wurde 16 h bei 4 °C gegen 4 l 50 mM Tris-HCl, 50 mM NaCl, 5 mM EDTA, pH 8 dialysiert, auf eine Salzkonzentration von 0,15 M NaCl, 50 mM Tris-HCl, 5 mM EDTA, pH 8 eingestellt und mit DNAase-freier RNAase (Endkonzentration 0,1 mg/ml) 30 min bei 37 °C inkubiert. Diese Mischung wurde wie oben beschrieben einmal mit Phenol/Chloroform/Isoamylalkohol und zweimal mit Chloroform extrahiert.

5 ml der so erhaltenen wäßrigen Lösung (DNA-Gehalt 30 µg/ml) wurden mit Natriumtetraborat auf eine Endkonzentration von 10 mM und mit festem Cäsiumchlorid (etwa 1,36 g/ml DNA-Lösung) auf einen Brechungsindex von 1.400 eingestellt. Nach Ultrazentrifugation bei 140.000 g (48 h, 20 °C) wurde der Inhalt des Zentrifugenröhrchens nach Punktion des Röhrchenbodens durch Austropfenlassen fraktioniert. Die DNA-haltigen Fraktionen wurden durch UV-Absorptionsmessung (260 nm) identifiziert und 16 h bei 4 °C gegen 4 l Lösung C (2 mM Tris-HCl, 2 mM NaCl, 0,1 mM EDTA, pH 8) dialysiert.

1.2 "Shotgun"-Klonierung in dem Cosmid-Vektor pHC79

Je 20 µg der gemäß 1.1 erhaltenen DNA wurden in 0,2 ml 100 mM Tris-HCl, pH 7,5, 50 mM NaCl, 10 mM $MgCl_2$ gelöst und mit 2,0 U BamHI jeweils 10, 20 und 30 min gespalten. Die Reaktionsansätze wurden vereinigt, durch Phenolbehandlung deproteinisiert und die DNA durch Zugabe von 2,5 Volumenteilen Ethanol 10 min bei -70 °C (Salzkonzentration: 0,3 M Natriumazetat, pH 5) durch Ethanolfällung konzentriert. Das Cosmid pHC79 (XI; Abb. 2) wurde durch Verdau mit 1,5 U BamHI/µg DNA in 100 mM Tris-HCl, pH 7,5, 50 mM NaCl, 10 mM $MgCl_2$, 6 mM DTT linearisiert, deproteinisiert und durch Ethanolfällung konzentriert. 7 µg der partiell mit BamHI gespaltenen DNA aus CBS 303.80 sowie 0,1 µg der BamHI gespaltenen DNA des Cosmids pHC79 wurden in 20 mM Tris-HCl, pH 7,5, 10 mM $MgCl_2$, 10 mM DTT, 0,6 mM ATP, 100 µg/ml Rinderserumalbumin bei einer DNA-Konzentration von 800 µg/ml mit 0,1 U T4-DNA Ligase 2 h bei 12 °C ligiert. Das Ligationsgemisch wurde in-vitro in Lambda-Phagenpartikel verpackt (XIV) und zur Infektion von E.coli HB 101 (XV) verwendet. Die infizierten Zellen wurden auf LB/Amp Agar (L-Broth: 1 % Bacto-Trypton, 0,5 % Hefeextrakt Difco, 0,5 % NaCl, 10 µg/ml Thiamin, 1 % Difco Agar, 100 µg/ml Ampicillin) ausgestrichen.

1.3 Herstellung eines Antikörpers gegen D-Hydantoinase

i. Herstellung reiner D-Hydantoinase

500 g Biotrockenmasse des D-Hydantoine spaltenden Stammes CBS 303.80 wurden in 5000 ml destilliertem Wasser suspendiert und unter Eiskühlung in einer Glaskugelmühle (Kugelgröße 0,35 bis 0,4 mm) bei 200 ml/min Pumpgeschwindigkeit aufgeschlossen. Anschließend wurde 20 %ige wäßrige Polymin P-Lösung bis zu einer Endkonzentration von 0,4 % zugegeben. Nach 15 min Zentrifugation bei 3000 g wurde der Zentrifugationsüberstand mit destilliertem Wasser verdünnt, bis die Leitfähigkeit 2 mSi betrug, und mit 230 g Whatman DE-52 Celluloseionenaustauscher gerührt (90 min, 22° C). Das gebundene Enzym wurde mit 0,1 M Borax-HCl, 0,2 M NaCl, pH 8,5 eluiert, entsalzt und auf einer DEAE A-50 Sephadex-Säule mit einem 0,1 bis 0,2 M NaCl-Gradienten in 50 mM Borax-HCl, pH 8,5 fraktioniert. Nach Dialyse des Eluats gegen 20 mM Borax-HCl, pH 8,5 wurde die Enzympräparation über eine AcA44 Ultrogelsäule (LKB) entsalzt und nach Einstellen auf 0,5 M Ammonsulfat durch hydrophobe Chromatographie an Octylsepharose CL4B (Pharmacia) weiter aufgetrennt. Der Hauptpeak war gemäß Analyse durch isoelektrische Fokussierung und SDS-Polyacrylamid Gelelektrophorese rein. Die Ausbeute betrug 10 %.

Die so erhaltene D-Hydantoinaselösung wurde gegen 0,1 M Borax-HCl, 0,1 M Natriumcitrat, pH 8,5 dialysiert und auf eine Kristallisierschale gegeben. Die Lösung wurde in einer geschlossenen Kammer über einer 0,5 M Natriumcitratlösung, pH 7,0, die nach 4 Tagen gegen eine 0,8 M Natriumcitratlösung, pH 7,0 ausgewechselt wurde, eingeengt. Nach 8 Tagen waren große Proteinkristalle mit D-Hydantoinase Aktivität entstanden.

In der aus dem Stamm CBS 303.80 erhaltenen D-Hydantoinase wurde die Aminosäure-Sequenz des Aminoterminus, interner tryptischer Peptide sowie des Carboxyterminus durch automatisierte Sequenzierung über Edman-Abbau bestimmt (XVI). Für das Protein wurden folgende Teilsequenzen ermittelt (XVII).

PLLIKNGEIITADSRYKADIYAEGXTIT(R)I-
(GQNLEAP) N-terminales Ende
TGPEWHEPS(RPXAV)
KGTIAVGSDADLVVY
TQHVNNDYNGFEGF
NFF$^{G}_{L}$.

ii) Gewinnung des Antikörpers

Drei Kaninchen wurden mit je 500 µg einer 0,9 %igen NaCl-Lösung der gemäß i) erhaltenen Enzympräparation in komplettem Freundschen Adjuvans (Difco) gespritzt und mit den halben Enzymmengen nach 6 und 10 Wochen nachgeimpft. 100 µl des nach der 10. Woche gewonnenen Antiserums banden 120 µg D-Hydantoinase. Die IgG-Fraktion des Kaninchenantiserums wurde durch Chromatographie an Protein A-Sepharose gewonnen.

1.4 Identifizierung D-Hydantoinase produzierender Zellklone

E.coli Kolonien, die ein bestimmtes Antigen produzieren (Empfindlichkeit ~ 1 pg (XVIII), lassen sich immunologisch identifizieren. Die einzelnen Schritte dieser Analyse bestehen in der Vorimprägnation von PVC-Folien mit einem Antikörper gegen D-Hydantoinase, Absättigung aller noch freien Bindungsstellen durch Präimmunserum, Kontaktabdruck der lysierten Kolonien und Bindung von radioaktiv markiertem Antikörper gegen D-Hydantoinase. Der radioaktive Antikörper bindet nur dort, wo vorher D-Hydantoinase oder Teilsequenzen davon an die Folie adsorbiert worden waren. Durch Autoradiographie der Folie mit Röntgenfilm wurden die Klone (Kolonien) identifiziert, die D-Hydantoinase oder Teilsequenzen des Enzyms synthetisieren:
Die gemäß 1.2 erhaltenen Klone (ca. 2000) wurden mit Zahnstochern von der Agarplatte in die Vertiefungen von Mikrotiterplatten abgeimpft, 16 h bei 37° C geschüttelt und im Raster der Mikrotiterplatten auf Nitrocellulosefilter (BA85, Schleicher & Schüll) auf LB/Amp-Platten überstempelt. Die etwa 2 mm großen Kolonien wurden mit 2,5 bis $5.10^5$ Lambda vir nin5 Phagen (XIX) in 10 mM $MgSO_4$ pro Kolonie infiziert und 4 h bei 37° C inkubiert. Anschließend wurden die Platten zur Vervollständigung der Zellyse für 10 min einer gesättigten Chloroform-Atmosphäre ausgesetzt. Daraufhin wurde eine mit der IgG-Fraktion des CBS 303.80 D-Hydantoinase Antikörpers behandelte PVC-Folie luftblasenfrei auf die lysierten Kolonien gelegt und über Nacht bei 4° C inkubiert. Die Folien waren in 0,2 M $NaHCO_3$, pH 9,2 mit 60 µg/ml IgG imprägniert (2 min, 20° C) und 2× mit 10 ml kaltem Waschpuffer (PBS-Puffer, 0,5 % Kaninchen Nullserum, v/v, 0,1 % Rinderserumalbumin, w/v) gewaschen. Die Folien wurden abgehoben, Zellreste vorsichtig entfernt und für 12 h in 2,5 ml Waschpuffer und $8.10^6$ cpm $^{125}$Iod markiertem anti-D-Hydantoinase CBS 303.80 IgG bei 4° C inkubiert. Die Markierung der IgG-Fraktion des Antikörpers gegen CBS 303.80 D-Hydantoinase (anti-D-Hydantoinase CB 303.80 IgG) erfolgte mit Hilfe von Chloramin T: 20 µl IgG in 50 mM Na-Phosphat, pH 7,5, 5 µl $Na^{125}I$ (Amersham-Buchler, 100 mCi/ml, 13-17 mCi/µg) wurden mit 10 µl Chloramin T (5 mg/ml in 50 mM Na-Phosphat, pH 7,5), 20 sec inkubiert, danach mit 10 µl $Na_2S_2O_5$ (12 mg/ml in 50 mM Na-Phosphat, pH 7,5), 100 µl KI (10 mg/ml) und 1 % Rinderserumalbumin versetzt und über Sephadex G50 (Pharmacia) entsalzt. Die Filter wurden nach Inkubation in 100 ml Waschpuffer 2×5 min bei 4° C und 2×5 min in $H_2O$ bei 4° C geschwenkt. Die getrockneten Folien wurden mit Verstärkerfolie und Kodak XAR5 Röntgenfilm über Nacht bei -70° C exponiert.

1.5 Nachweis des von den Klonen gebildeten D-Hydantoins

Die Zellklone, die im oben beschriebenen Immunassay (Broome-Gilbert XVIII) positiv indentifiziert worden waren, wurden auf Expression enzymatisch aktiver D-Hydantoinase geprüft. 50 ml-Kulturen wurden dazu in L-Broth, die 100 µg/ml Ampicillin enthielt, 16 bis 24 h bei 37° C geschüttelt und danach 10 min bei 4000 bis 6000 g zentrifugiert. Der Niederschlag wurde in 50 ml Lösung A resuspendiert, abzentrifugiert, in 130 ml Lösung A aufgenommen und mit 20 µl Hühnereiweiß-Lysozym-Lösung (10 mg/ml in 25 mM Tris-HCl, pH 8) 20 min bei Raumtemperatur inkubiert. 330 µl 0,25 M EDTA-Lösung, pH 8 wurden zugegeben. Nach 5 min wurden unter raschem Mischen 1,67 ml Lösung B zugesetzt. Das klare Lysat wurde 1 h hochtourig bei 4° C abzentrifugiert, der Überstand 2 min auf 70° C erhitzt und 10 min bei 4° C und 4000 g vom Präzipitat befreit. Zu 2 ml des Überstands wurden 0,5 ml 0,4 M Borax-HCl, pH 8,5, 0,1 ml 1 M $MgCl_2$ und 0,625 ml 5 %ige Methylthioethylhydantoin-Lösung in 0,1 M Borax-HCl, pH 8,5 gegeben. Nach 30 min Schütteln bei 60° C (zur Umsetzung von D,L-Methylthioethylhydantoin zu N-Carbamoyl-D-methionin) wurden 1,5 ml entnommen, 5 min bei 8000 g zentrifugiert und zu 1,2 ml des Überstandes 0,168 ml 1/10 konzentrierte Phoshporsäure gegeben. Das sich bildende Präzipitat wurde 1 min bei 8000 g abzentrifugiert, der Überstand partikelfrei filtriert und durch HPLC auf Gehalt an N-Carbamoyl-methionin analysiert. Die spontane Umwandlung von D,L-Methylthioethylhydantoin zu N-Carbamoyl-D-methionin unter diesen Bedingungen wurde in Gegenwart einer gleichgroßen Menge Proteinextrakt aus E.coli HB101 (pHC79) bestimmt. Klone, deren Lysate mindestens die doppelte Umwandlungsrate von D,L-Methylthioethylhydantoin zu N-Carbamoyl-D-methionin aufwiesen, wurden als positiv gewertet.

1.6 Subklonierung des D-Hydantoinase-Gens

Die rekombinante Plasmid-DNA eines der D-Hydantoinase produzierenden Klone (Bam7B10; Konstruktion siehe Fig. 2) wurde nach bekannten Methoden (XX) isoliert. 2 µg Plasmid-DNA wurden in einer Konzentration von 120 µg/ml mit 0,5 U Sau3A pro µg DNA 10, 20 bzw. 30 min gespalten. Die Reaktionsansätze wurden vereinigt und auf einem 0,8 %igen Soft-Agarose Gel in einem 50 mM Tris-Azetat Puffer, pH 7,8, welcher 2 mM EDTA und 0,5 µg/ml Ethidiumbromid enthielt, elektrophoretisch aufgetrennt. Das Gelsegment, das DNA in einem Größenbereich von 2 bis 4 Kilobasen enthielt, wurde mit Hilfe einer UV-Lampe durch Vergleich mit einem DNA-Größenstandard identifiziert, abgetrennt und mit demselben Volumen Lösung D (0,1 M Tris-HCl, 10 mM EDTA, 0,2 M NaCl, pH 7,4) 5 min bei 65° C aufgeschmolzen. Nach Emulsion mit demselben Volumen 37° C warmem wäßrigen Phenols (äquilibriert mit Lösung D/Wasser = 1:1) und Zentrifugation wurde die entstehende Oberphase noch zweimal mit Chloroform extrahiert und durch eine Ethanolfällung konzentriert. 1 µg Plasmid pBR327 (X) wurde mit 1,5 U/µg DNA BamHI 90 min linearisiert und mit Kälberdarmphosphatase (CIP) dephosphoryliert (XXI). 0,5 µg der durch Phenolextraktion deproteinisierten Plasmid-DNA wurden mit 1 µg der 2-4 kb-Fraktion von Bam7B10 ligiert (XXII). Mit dem Ligationsansatz wurden $CaCl_2$-behandelte (XXIII) E.coli HB101-Zellen (XV) transformiert. Nach 90 min Wachstum in antibiotikafreiem Nährmedium (L-Broth: 1 % Bacto-Trypton, 0,5 % Hefeextrakt Difco, 0,5 % NaCl, 10 µg/ml Thiamin) wurden die Zellen auf LB/Amp-Platten ausplattiert. Die gewonnenen Klone wurden wie oben beschrieben im Broome-Gilbert-Assay sowie durch Nachweis gebildeten Hydantoins auf D-Hydantoinase positive Klone geprüft. 3 Klone bildeten enzymatisch aktive D-Hydantoinase. Einer von ihnen, B6 (siehe Fig. 2), wurde nach Sequenzieren für die Expressionsoptimierung verwendet.

1.7 Sequenzierung des CBS 303.80 D-Hydantoinase Gens im Vektor B6

Das CBS 303.80 DNA-Fragment, das im Vektor B6 kloniert vorlag, enthielt keine Schnittstellen für die Restriktionsendonukleasen BamHI, ClaI, EcoRI, HindIII, PstI, PvuII, SalI, KpnI, HpaI, SalI und XbaI. Eine singuläre HindII-Stelle im klonierten DNA-Fragment wurde in eine BamHI-Stelle durch BamHI Linkerintegration (5′pCCGGATCCGG-3′) in partiell HindII geschnittenes Plasmid B6 (Fig. 3) umgewandelt. Eines der resultierenden Plasmide (pBGA, Fig. 4) führte nicht mehr zur Expression von D-Hydantoinase. Folglich sollte die BamHI Linkerintegration im Genbereich stattgefunden haben. Deshalb wurde von der BamHI-Schnittstelle aus nach 5′-$^{32}P$ Markierung nach beiden Seiten sequenziert (XXIV). Nach Kinasierung wurde mit EcoRI der Sekundärschnitt durchgeführt und die zwei zu sequenzierenden Fragmente über niedrig schmelzende Agarose gereinigt. Aus den erhaltenen Sequenzdaten resultierte die Position und die Orientierung des D-Hydantoinase-Gens. Abwärts der BamHI-Stelle liegt nach 225 Nukleotiden das Stopcodon. Das Leseraster konnte exakt zugeordnet werden, da das sequenzierte Fragment über 10 Aminosäurereste exakt mit einem sequenzierten tryptischen Fragment korrelierte (NNDYNGFEGF; vgl. Beispiel 1.3i). Mit Hilfe überlappender Deletionsmutanten (XXV) wurde die Sequenzierung des D-Hydantoinase-Gens gezielt durchgeführt.

i) Kinasierung der Linker

BamHI Linker (5'-CCGGATCCGG-3') und HindIII Linker (5'-GCAAGCTTGC-3') wurden in 20 µl Reaktionsansatz in einer Menge von 250 pMol 30 min bei 37° C in 60 mM Tris-HCl, pH 7,5, 10 mM $MgCl_2$, 15 mM DTT, 1 mM Spermin, 100 µM ATP, 8 U T4 Polynukleotidkinase (PL Biochemicals) in die 5'-phosphorylierten Formen überführt. Die Ansätze wurden anschließend eingefroren und Aliquots direkt für die Ligierung eingesetzt (s.u.).

ii) BamHI Linkerintegration in partiell HindII geschnittenes Plasmid B6

4 µg Plasmid B6, das in bekannter Weise isoliert wurde, wurde mit 1 U HindII (Boehringer, Mannheim) 10 min bei 37° C verdaut. Nach Phenolextraktion und Konzentrierung durch Ethanolpräzipitation wurde in einem 50 µl Ansatz die BamHI Linkerintegration in einem etwa 50fachen Überschuß des Linkers bezogen auf die freien Enden des Plasmids durchgeführt (100 pMol Linker, 2 pMol Enden ≙ 4 µg B6, mit HindII partiell geschnitten, 66 mM Tris-HCl, pH 7,5, 6,6 mM $MgCl_2$, 10 mM DTT, 0,4 mM ATP, 5 U T4 DNA-Ligase; 14 h, 15° C). Nach Phenolextraktion und Ethanolfällung folgte vollständiger Verdau der ligierten DNA in 40 µl mit 10 U BamHI. Das Reaktionsgemisch wurde auf einem 1 % Agarosegel (niedrig schmelzende Agarose, Biorad) aufgetrennt. Lineares Plasmid wurde durch Phenolextraktion des geschmolzenen Gelsegments eluiert und nach Reinigung über DE52 (Whatman; Bindung in 0,15 M NaCl, 10 mM Tris-HCl, pH 7,5, 1 mM EDTA, Elution mit 1 M NaCl, 10 mM Tris-HCl, pH 7,5, 1 mM EDTA) durch Ethanolfällung konzentriert. Die Ausbeute betrug etwa 1 µg. 100 ng DNA wurden in 20 µl Ligationsansatz (66 mM Tris-HCl, pH 7,5, 6,6 mM $MgCl_2$, 10 mM DTT, 0,4 mM ATP; 14 h, 15° C) mit 0,5 U T4 DNA-Ligase zyklisiert und in $CaCl_2$-behandelte E.coli HB101-Zellen transfektiert. Rekombinante Klone wurden in bekannter Weise selektiert. Die möglichen rekombinanten Plasmide pB6A, pB6B, pB6C (Abb. 4) wurden über EcoRI/BamHI Doppelverdau zugeordnet. Das Plasmid pB6A wurde für die Sequenzierung (XXIV) eingesetzt, wobei von der BamHI-Stelle nach beiden Seiten sequenziert wurde (siehe Fig. 4). Das Plasmid pB6B diente als Ausgangsplasmid zur Erzeugung von Delta 5' und Delta 3'-Mutanten, die wie folgt dargestellt wurden.

iii) B6 Delta 5'-Mutanten

7,5 µg pB6B (Fig. 4), linearisiert mit BamHI wurden in 100 µl 12 mM $CaCl_2$, 12 mM $MgCl_2$, 600 mM NaCl, 20 mM Tris-HCl, pH 8,0, 1 mM EDTA mit 2,4 U Bal31 (BRL) bei 25° C inkubiert. Nach 4 und 6 min wurden je 16 µl entnommen. Die Proben wurden sofort phenolisiert und vereinigt. Entsprechend wurden zwei Proben nach 9 und 11 min bzw. 14 und 16 min behandelt. Nach Chloroform Extraktion und Konzentrierung durch Ethanolfällung wurden die Enden der DNA mit Kornberg-Polymerase (PolI) bündig geschnitten, um anschließend einen Linker anzufügen. Die PolI Behandlung wurde in 100 µl 120 mM $K\text{-}PO_4$, pH 6,9, 6 mM $MgCl_2$, 1 mM dATP, 1 mM dTTP, 1 mM dGTP, 1 mM dCTP, 5 mM DTT, 1,5 µl alpha$^{32P}$ dATP, 400 Ci/mmol, 10 mCi/ml, 10 U DNA-Polymerase I (Boehringer, Mannheim) mit 2 µg Bal31 behandelter DNA bei 15° C für 2 h durchgeführt. Die Reaktionsansätze wurden dann über Sephadex G50 (Pharmacia) in 100 mM NaCl, 50 mM Tris-HCl, pH 7,5, 5 mM EDTA fraktioniert. Die DNA des Ausschlußvolumens wurde in 0,15 M NaCl, 10 mM Tris-HCl, pH 7,5, 1 mM EDTA an DE52 (Whatman) gebunden, mit 1 M NaCl, 10 mM Tris-HCl, pH 7,5, 1 mM EDTA eluiert und mit Ethanol gefällt. Die anschließende BamHI Linkerintegration wurde wie oben beschrieben in 30 µl durchgeführt. Nach Phenol- und Chloroformextraktion sowie Konzentrierung durch Ethanolfällung wurden die Proben mit BamHI verdaut und in bekannter Weise auf 0,8 % niedrig schmelzender Agarose (Gelkonzentration 0,8 %) aufgetrennt und gereinigt. Die so gereinigte DNA wurde in 20 µl Ansätzen mit 0,5 U T4 DNA-Ligase religiert (12 h, 12° C). Mit je 10 µl Ligationsansatz wurden $CaCl_2$ behandelte E.coli-HB101-Zellen transfektiert, und die resultierenden Rekombinanten wurden auf bekannte Weise selektiert. Von den 4 bis 6 min bzw. 9 bis 11 min Bal31-Behandlungen wurden je 15 Plasmide isoliert und charakterisiert. Die Plasmide Delta B6 5'A, -B, -E, -I und -K wurden von der BamHI-Schnittstelle aus nach XXIV sequenziert wie oben beschrieben (Sekundärspaltung mit EcoRI (vgl. Fig. 5).

iv) B6 Delta 3'-Mutanten

Die 3'-Mutanten wurden auf die gleiche Art erzeugt wie die 5'-Mutanten.

Als Ausgangs-DNA diente pB6B (vgl. Fig. 4) nach Linearisierung mit SmaI (Abb. 5). 10 µg wurden in einem Reaktionsvolumen von 33 µl mit 1,4 U Bal31 in der oben angegebenen Pufferkonzentration inkubiert. Nach jeweils 2 bzw. 4, 6 bzw. 8, 10 bzw. 12, 14 bzw. 16 und 18 bzw. 20 min wurden 3 µl phenolisiert und paarweise aufgearbeitet. Nach PolI-Reparatur wurde der HindIII-Linker in die Plasmid-DNA integriert und diese nach Verdau mit HindIII und Religation unter bekannten Bedingungen in HB101-Zellen transfektiert. Nach Plasmidcharakterisierung von 48 Rekombinantenplasmiden (2 bis 12 min Bal31-Behandlung) wurden die 3'-Deletionsmutanten O, P, R und S erhalten. Die Position der HindIII-Schnittstelle wurde über Restriktionskartierung bzw. Sequenzierung festgelegt. Die Sequenzierung erfolgte von der HindIII-Stelle aus mit EcoRI als Sekundärschnitt nach XXIV.

Die mit den unter 1.7 ii-iv beschriebenen Schritten erhaltenen Sequenzdaten sind in Fig. 6 zusammengefaßt. Der kodierende Bereich des D-Hydantoinasegens erstreckt sich von Nukleotid 327 bis Nukleotid 1435. Die drei Nukleotide, die den Start der Translation signalisieren und das Translationsstopsignal sind durch Einrahmung gekennzeichnet. Innerhalb der Sequenz entspricht der Bereich 1 einer Länge von 250 ± 50 Nukleotiden, der Bereich 3 90 ± 1 Nukleotiden. Bereich 2 entspricht eine Sequenz NN oder NNN. (N=A,G,C oder T). Die entsprechenden Bereiche sind eingerahmt.

1.8 Expressionsoptimierung

Das Rekombinanten-Plasmid Delta B6 5'E enthält noch ca. 250 Nukleotide CBS 303.80 DNA vor dem Translationsstartkodon des D-Hydantoinse-Gens, nachdem die früher im Plasmid pB6b vorhandene SalI-Erkennungsstelle unter Deletion der benachbarten 200 bis 300 Nukleotide durch eine BamHI-Schnittstelle ersetzt worden war (vgl. 1.7 iii).

E. coli HB 101 Zellen, die mit diesem Plasmid transformiert wurden exprimieren enzymatisch aktive D-Hydrantoinase. In gleicher Weise wurde, ausgehend von der in der 3'-flankierenden DNA-Sequenz liegenden SmaI-Schnittstelle, die SmaI Schnittstelle durch eine HindIII-Schnittstelle ersetzt (Fig. 5 und 1.7 iv). Die begleitende CBS 303.80-DNA-Sequenz wurde dabei bis auf 25 Nukleotide hinter dem Translationsstopkodon des D-Hydantoinase Gens eliminiert (Klon Delta B6 3'O). Durch Übertragung des HindII-Fragments aus dem Plasmid Delta B6 3'O in das Plasmid Delta B6 5'E wurde ein neues Plasmid konstruiert, das vor dem Hydantoinase-Gen noch ca. 250 Nukleotide, nach dem Stop-Kodon des Gens noch 25 Nukleotide Fremd DNA aufweist (Delta B6 EO, Fig. 7). Eine weitere Deletion der ca. 250 Nukleotide Fremd-DNA vor dem Hydantoinase Gen erwies sich in der Plasmidkonstruktion Delta B6 EO als nicht durchführbar; möglicherweise weil bei weiteren Bal-Deletionen der Replikationsursprung des Vektors (pBR327) beeinträchtigt wurde. Um weitere Deletionen einzuführen, wurde das D-Hydantoinase Gen aus Delta B6 EO in pBR322 als BamHI/HindIII Fragment in bekannter Weise umkloniert. Das resultierende Plasmid pBR EO (Fig. 7) wurde anschließend wie oben beschrieben nach Linearisierung durch BamH1 mit Bal31 verkürzt. Nach Beendigung des Bal31-Verdaues, Reparatur der Enden mit T4-DNA-Polymerase und Einfügen des BamHI-Linkers wurden nach Transformation von E.coli HB101 8 Klone selektiert, deren Plasmid-DNA nach BamHI/HindII-Verdau ein D-Hydantoinase Genfragment im geeigneten Größenbereich von 1,5 bis 1,3 kb aufwiesen. Das D-Hydantoinase Gen wurde aus diesen Plasmiden nach Isolierung der DNA auf bekannte Weise durch BamHI/HindIII-Verdau herausgeschnitten und in die Plasmide pEX31a, b bzw. c integriert, wobei die Empfängerplasmide vorher ebenfalls mit BamHI und HindIII verdaut worden waren. Die Plasmide pEX31a-c leiten sich von dem Plasmid pPLC24 (XXVI) ab und enthalten den temperaturinduzierbaren Lambdapromotor pl sowie nach 97 Aminosäurekodons der MS2 Replikase eine Polylinkerregion, die in den Konstruktionen a-c die Ankoppelung eines Fremdgens an die MS2 Replikase in allen drei Leserahmen gestattet (Fig. 8). Die Ligationsprodukte wurden in den E.coli Stamm N4830 (PL Biochemicals) transformiert, der das Gen für den temperaturlabilen Lambdarepressor cI 857 enthält. Ampicillin resistente Transformanten wurden durch Inkubation bei 28°C erhalten. Klone, die nach Hitzeinduktion D-Hydantoinase bildeten, wurden zunächst immunologisch nachgewiesen.

Dazu wurden Einzelkolonien in 150 µl LB/Amp Medium in den Boden von U-Boden Mikrotiterplatten abgeimpft, über Nacht bei 28°C geschüttelt, in frisches Medium (120 µl) in 1:3-Verdünnung überimpft und in einer Mikrotiterplatte bei 42°C 3 h unter Schütteln inkubiert. Nach Zugabe von 20 µl 10 % SDS-Lösung wurde die Mikrotiterplatte mit Deckfolie versiegelt und 1 h auf der Oberfläche eines 50°C Wasserbads zur vollständigen Lyse der Bakterien inkubiert. Die Lysate wurden dann über eine geeignete Vorrichtung (Hybridot Manifold 1050 MM der Firma BRL) auf ein Nitrocelluloseblatt (BA85, Schleicher & Schuell) abgesaugt und nach Waschen mit PBS-Puffer in beschriebener Weise (XXVII) mit einer 1:150-Verdünnung eines D-Hydantoinase Antikörpers (IgG-Fraktion eines Kaninchen Antiserums) und anschließend nach Auswaschen des ersten Antikörpers mit Meerrettich Peroxidase gekoppeltem Anti-Kaninchen IgG-Antikörper und Farbnachweis der gebundenen Meerrettich Peroxidase mittels o-Dianisidin/$H_2O_2$ behandelt. 8 dieser Klone zeigten bei der Umsetzung von Methylthioethylhydantoin enzymatische Aktivität, unabhängig davon in welchen Leserahmen der Plasmide pEX31a-c die D-Hydantoinase Genfragmente einkloniert worden waren. Die Expression der Enzymaktivität war temperaturinduzierbar. Die Klone IF10, 3A8 und IID2 zeigten die höchsten Enzymaktivitäten. Sie lag um den Faktor 40 höher als im Ausgangsstamm bezogen auf Biofeuchtmasse bei Fermentation in folgendem Medium: 5 g Hefeextrakt, 2,5 g Trypton, 10 g Nutrient Broth, 0,258 g $Na_3PO_4 \times 12\ H_2O$, 10 ml Castenholz-Basalmedium pro l. Die Zellen wurden bis $A_{600}$ = 0,3 bei 30°C inkubiert, auf 45°C 15 min erhitzt und 5 h bei 37° C fermentiert. Die Zellen wurden nach Zentrifugation in Lösung A + 1 mM $MnCl_2$ gewaschen und in diesem Puffer direkt zur Umwandlung der Hydantoine eingesetzt.

In den von den Plasmiden pEX31a, b und c abgeleiteter Expressionsvektoren IF10, 3A8 und IIDI, die CBS 303.80 D-Hydantoinase exprimieren, wurde der Übergang von den Vektorsequenzen zum D-Hydantoinasegen durch Sequenzierung identifiziert. Der Übergang ist jeweils mit einem Pfeil und dem Namen der entsprechenden Konstruktion in Fig. 6 eingezeichnet. Der Übergang erfolgt über einen BamH1 Linker an der Basis der Pfeile, die Linkersequenz ist in der Fig. 6 nicht eingezeichnet.

1.9 Charakterisierung der D-Hydantoinase CSB 303.80 aus rekombinanten E.coli-Klonen

Zur molekularen Charakterisierung der D-Hydantoinase aus gentechnologisch modifizierten E.coli-Stämmen wurde eine 500 ml-Kultur HB101 mit dem Plasmid B6 16 h bei 37° C in LB/Amp unter Schütteln inkubiert und wie unter 1.5 beschrieben zur Gewinnung enzymatisch aktiver D-Hydantoinase aufgearbeitet, wobei die eingesetzten Puffermengen um den Faktor 10 erhöht wurden. Nach hochtouriger Zentrifugation des klaren Lysats für 1 h bei 4° C wurde der Überstand auf 0,1 % Polymin P eingestellt, 2 min auf 70° C erhitzt und 15 min niedertourig zentrifugiert. Der Überstand wurde 5fach in einer Amicon-Druckzelle konzentriert. 30 µl des Konzentrats wurden auf ein isoelektrisches Fokussierungsgel aufgetragen (pH 3,5 bis 9,5; Ampholine PAGE-Platte LKB). Reine D-Hydantoinase aus CBS 303.80 wurde parallel als Kontrolle aufgetragen. Nach Beendigung der isoelektrischen Fokussierung wurden beide Trennbahnen in 3 mm Segmente geschnitten. Die Gelsegmente wurden direkt für den Test auf enzymatisch aktive D-Hydantoinase durch Umwandlung von Methylthioethylhydantoin in N-Carbamoylmethionin wie oben beschrieben eingesetzt. Die D-Hydantoinasen aus E.coli HB101 (B6) und CBS 303.80 hatten denselben isoelektrischen Punkt.

4 l einer Kultur von E.coli HB101 (B6) wurden wie oben beschrieben zu einem klaren Lysat aufgearbeitet. Nach 60 min hochtouriger Zentrifugation bei 4° C wurde der Zentrifugationsüberstand auf 0,4 % Polymin P eingestellt, 2 min auf 70° C erhitzt und 15 min mitteltourig zentrifugiert. 165 ml des Überstandes wurden über eine Affinitätssäule chromatographiert, die durch Bindung von 1 ml Kaninchen-D-Hydantoinase-Antiserum an 1 g Bromcyan-aktivierte Sepharose hergestellt worden war (XXVIII). 5 µg der eluierten D-Hydantoinase wurden auf einem SDS-Gel (5 % Sammelgel, pH 6,8, 10 % Trenngel, pH 8,8) in Tris-Glycin Puffer (XXIX) aufgetrennt. Als Kontrolle wurde authentische D-Hydantoinase aus CBS 303.80 aufgetrennt. Die Proteinbanden wurden durch Anfärben mit Coomassie Brilliant Blue sichtbar gemacht. D-Hydantoinase aus E.coli und CBS 303.80 wanderten gleichschnell.

Beispiel 2

Shotgun-Klonierung des Hydantoinase-Gens aus Lu1220

2.0 Isolierung thermophiler Bacillacaeen

Thermophile, aerobe, sporenbildende Bakterien mit Hydantoinaseaktivität lassen sich nach folgendem Verfahren aus Bodenproben, Pflanzenmaterial (insbesondere Kompost) und Wasserproben (vorzugsweise nährstoffhaltige Wässer) isolieren (vgl. auch I):
Eine Spatelspitze der Probe wird in Nährlösung oder Saline (0,85 % NaCl) suspendiert. Wasserproben werden direkt untersucht, wobei das Probenvolumen ca. 10 ml beträgt. Die Probe wird durch Erhitzen auf 80° C für 10 min pasteurisiert. Aliquots der pasteurisierten Probe werden in bekannter Weise dezimal verdünnt und auf Nähragar ausgestrichen. Die Schalen mit Nähragar werden in Plastikfolie verpackt und 1 bis 4 Tage bei 60° C bebrütet. Die sich entwickelnden Kolonien werden abgeimpft und durch wiederholtes Übertragen auf Agarkulturen gereinigt. Die erhaltenen Reinkulturen werden auf Hydantoinaseaktivität geprüft.

Ein so erhaltener Stamm mit der internen Bezeichnung LU 1220 wurde für das Beispiel verwendet.

2.1 Präparation der DNA

200 ml Castenholz-Medium wurden mit einer Kolonie Lu 1220 angeimpft und unter Schütteln (160 rpm) bei 60° C bis 190 Klett-Einheiten kultiviert. Nach Zentrifugation bei 4000 rpm, 20 min, 4° C (Minifuge Heraeus) wurde das Naßgewicht mit 1,16 g bestimmt. Der Zentrifugationsrückstand wurde mit 25 ml 150 mM NaCl, 100 mM EDTA, pH 8,0 gewaschen und nochmals pelletiert. Nach Aufnahme der Zellen in 15 ml 150 mM NaCl, 100 mM EDTA, pH 8,0 und Zugabe von 0,5 ml Lysozym (Sigma; 10 mg/ml in 150 mM NaCl, 100 mM EDTA, pH 8,0) wurden die Zellen 60 min bei 37° C inkubiert, daraufhin mit 1 ml 25 % wäßriger SDS-Lösung versetzt und 10 min bei 60° C inkubiert (Lysis der Zellen). Bei 20° C wurden 0,25 Vol. 5 M $NaClO_4$ zugesetzt. Die weitere Aufarbeitung erfolgte wie in Beispiel 1.1 bis 1.2 dargelegt. Die DNA wurde schließlich in 150 µl 20 mM Tris-HCl, pH 8,0 aufgenommen. Die Endreinigung wurde über CsCl-Gradientenzentrifugation durchgeführt. Einer CsCl-Lösung (7 ml $H_2O$, 0,11 ml 100 mM $Na_2B_4O_7$, 1,36 g CsCl (n = 1.400)) wurden 75 µl DNA-Lösung überschichtet, im TST 41 Rotor (Kontron Ultrazentrifuge) bei 20° C, 30 h hochtourig zentriguiert und die DNA aus 2 von 15 0,5 ml-Fraktionen gesammelt. Die DNA wurde nach Zugabe von 3 ml $H_2O$ und 12 ml Ethanol bei 0° C gefällt, 2× mit 80 % Ethanol gewaschen und in 100 µl 20 mM Tris-HCl, pH 8,0 gelöst. Die Ausbeute betrug 120 µg DNA.

2.2 Cosmid-Shotgun-Klonierung

Lu1220 DNA wurde mit Sau3A partiell geschnitten (20 µg DNA mit 1 bzw. 3 U Sau3A 1 h bei 37° C). Durch Elektrophorese in 0,8 % Agarosegelen wurde sichergestellt, daß die durchschnittliche Fragmentgröße >20 kb betrug. Die Fragmente wurden, wie in Beispiel 1 beschrieben, in die BamHI Erkennungsstelle von pHC79 ligiert, in vitro verpackt und über E.coli HB101 in eine Cosmid-Genbank überführt. 1.600 Kolonien wurden einzeln in 100 µl LB/Amp (Kulturen auf Mikrotiterplatten) in Duplikat überstempelt. Jeweils eine Platte wurde als Glyzerinkultur (20 % Glyzerin) bei -70° C gelagert.

2.3 Identifizierung der D-Hydantoinase Lu1220 positiven Cosmid-Klone mit Hilfe eines immunologischen Assays.

Für die D-Hydantoinase aus Lu1220 stand ein polyklonaler Antikörper zur Verfügung (Gewinnung wie in Beispiel 1 für polyklonalen Antikörper gegen CBS 303.80 beschrieben), mit dem es wie in Beispiel 1 beschrieben möglich war, die D-Hydantoinase, die unter eigener Promotoraktivität in E.coli produziert wurde, koloniespezifisch nachzuweisen. Dazu wurden 1500 Klone wie in Beispiel 1 beschrieben gemäß XVIII auf Expression von Lu1220 D-Hydantoinase Sequenzen ausgetestet. 8 Kolonien erwiesen sich als anti-D-Hydantoinase Lu1220 IgG Antigen-positiv. Alle Klone wurden auf enzymatische Aktivität getestet (150 ml Kulturen in LB/Amp, 1 mM $MnCl_2$). Die Kulturen wurden bis ~300 Klett-Einheiten bei 37° C gezogen, die Zellen 20 min bei 4° C, mitteltourig abzentrifugiert ("Minifuge", Heraeus) das Pellet mit 15 ml Lösung A (50 mM Tris-HCl, pH 8,0, 10 % Saccharose), 1 mM $MnCl_2$ gewaschen, resuspendiert mit 1,3 ml Lösung A, 1 mM $MnCl_2$ und mit 270 µl Lysozym (10 mg/ml) in Lösung A) versetzt, 20 min bei 20° C inkubiert. 330 µl 0,25 M EDTA, pH 8,0 wurden zugesetzt sowie nach 5 min Inkubation bei 20° C mit 1,67 ml Lösung B sofort gemischt und 1 h bei 4° C, hochtourig zentrifugiert. Zu je 2 ml Überstand wurden 0,5 ml Borax-Puffer, pH 8,15, 0,13 ml 1 M $MgCl_2$ und 0,5 ml 5 % Methylthioethylhydantoin zugegeben und 4 h bei 60° C geschüttelt. 1,5 ml dieser Ansätze wurden 5 min in einer Eppendorf-Tischzentrifuge zentrifugiert. 1,2 ml des Zentrifugationsüberstandes wurden mit 0,168 ml 1:10 verdünnter Phosphorsäure versetzt, steril filtriert und über HPLC analysiert. Eine Kultur (C5, vgl. Fig. 9) erwies sich als Produzent enzymatisch aktiver D-Hydantoinase.

2.4 Plasmidsubklonierung in pBR327

Aus einer 100 ml Kultur von C5 in LB/Amp wurde Plasmid-DNA präpariert (vgl. Beispiel 1). 10 µg C5-DNA wurden in 100 µl mit 1,5 U Sau3A 1 h bei 37° C geschnitten und direkt auf 0,8 %ige niedrig schmelzende Agarose (Biorad) aufgetragen. Die DNAs mit einer Größe von 1.500 bis 3.000 Nukleotiden wurden aus dem Gel eluiert, gefällt und gelöst in 20 µl 20 mM Tris-HCl, pH 8,0, 0,1 mM EDTA. 8 µl (200 ng) partiell Sau3A-geschnittene C5-DNA wurden in 25 µl mit einer äquimolekularen Menge pBR327 ligiert (1U T4 DNA-Ligase, NEN, 15° C über Nacht). Der pBR327-Vektor war mit BamHI geschnitten und mit Kälberdarmphosphatase (Boehringer, Mannheim) 5′ dephosphoryliert worden, um Selbstligation des Vektors zu unterdrücken (XXX).

10 µl des Ligationsansatzes wurden in E.coli HB101 transformiert (XXXI) und die Transformationsansätze auf LB/Amp-Platten plattiert. Es resultierten 500 Kolonien, wie für die Cosmid-Klone beschrieben, in Mikrotiterplatten überführt und wiederum nach XVIII auf D-Hydantoinase-Antigen getestet wurden. 4 Klone zeigten produziertes Antigen an. aus 100 ml LB/Amp-Kultur dieser 4 Vertreter wurde Plasmid-DNA präpariert, mit je 50 ml wurden wie oben beschrieben die Enzymteste auf D-Hydantoinase durchgeführt. 2 Klone zeigten enzymatische Aktivität. Die Plasmide dieser Klone (F2 und B4) waren offensichtlich identisch und hatten unerwartet große DNA-Fragmente integriert (~8 kb), während zwei weitere ein 1,9 kb bzw. ein ~2.1 kb langes Fragment integriert enthielten.

Plasmid-DNA des Klons F2 wurde für eine weitere Subklonierung herangezogen, um einen Klon zu erhalten, der eine möglichst kleine Insertlänge aufweist und dennoch die Produktion enzymatisch aktiver D-Hydantoinase bewirkt:

10 µg F2-DNA wurden in 100 µl mit 3 U Sau3A 1 h verdaut, auf 0,8 % niedrig schmelzender Agarose wie oben beschrieben größenfraktioniert (1.500 bis 3.000 bp). Die Fragmente wurden wie beschrieben in die BamHI-Stelle von pBR327 integriert und in HB101 transformiert. Es wurden 650 Kolonien erhalten, davon reagierten im Broome-Gilbert Assay (XVIII) 23 Kolonien antigenpositiv, 4 davon zeigten sehr starke Signale, 8 Kolonien wiesen sich als Produzenten aktiver D-Hydantoinase (z.B. A59 und E64). Als Klon mit der höchsten Produktivität erwies sich A59 mit einer Insertlänge von 1.734 bp (Fig. 9).

A59 wurde zur Expressionsoptimierung, A59 und E64 (Fig. 10) wurden zur Sequenzanalyse des D-Hydantoinase-Gens herangezogen.

2.5 Sequenzierung

Die Sequenzierung wurde nach der in Fig. 11 dargestellten Strategie durchgeführt. Es wurden nicht nur die natürlichen Restriktionsstellen zur Sequenzierung herangezogen, sondern auch künstliche Restriktionsstellen, die nach der Methode von Frischauf et al. (XXV) in die Plasmide A59 und E64 eingeführt wurden. Das Ziel war, eine Serie einseitiger, durch Linkerschnittstellen begrenzter (Delta 5') Deletionsmutanten zu erhalten, so daß die Sequenzierung der Fragmente zu einer kompletten, überlappenden Sequenz des Inserts führt (Fig. 11. Der Pfeil am Hydantoinsegen bezeichnet die Leserichtung, der schwarz unterlegte Teil des Inserts die rodierende Region.). Die Sequenzierung wurde nach XXIV durchgeführt.

40 µg Plasmid-DNA A59 wurden in 250 µl DNAase I Puffer (20 mM Tris-HCl, pH 7,5, 1,5 mM $MnCl_2$, 100 µg/ml Gelatine) mit 200 pg DNAase (Worthington, Stammlösung: 1 mg DNAase in 1 ml 50 % Glyzerin, 150 mM Na-Acetat, pH 5,0, 1 M NaCl, 0,5 mg/ml Gelatine) 6 min bei 24°C verdaut, mit 5 µl 0,5 M EDTA, pH 8,0 wurde die Reaktion gestoppt, mit 250 µl Phenol (mit Puffer äquilibriert) ausgeschüttelt, chloroformiert, ausgeethert und auf einem 0,8 % niedrig-schmelzenden Agarosegel elektrophoretisch aufgetrennt (100 V/20 cm, 2 h). Die Bande des linearisierten Plasmids wurde ausgeschnitten, die DNA eluiert, über DE52-Cellulose (Whatman) gereinigt und in 20 µl 20 mM Tris-HCl, pH 7,5, 0,1 mM EDTA gelöst (~5 µg DNA). Die Enden der DNA wurden mit 5 U Polymerase I Klenow-Fragment (Boehringer, Mannheim) bündig gemacht durch 30 min Inkubation bei 20°C (XXXII). Daraufhin wurde an 1,5 µg linearisierter A59DNA (~0,8 pMol Enden) im 50-fach molaren Überschuß kinasierte HindIII-Linker (5'-GCAAGCTTGC-3'; P&L) ligiert in 25 µl Ligationsansatz (XXII) in Gegenwart von 10 U T4 DNA-Ligase (NEN). Nach Inkubation über Nacht bei 15°C wurde die Ligase 10 min bei 65°C inaktiviert. 25 µl Y100-Puffer (100 mM NaCl, 10 mM Tris-HCl, pH 7,5, 6 mM $MgCl_2$, 1 mg/ml Gelatine, 6 mM β-MSH) wurden zugesetzt, und die DNA wurde mit 5 U HindIII (Boehringer, Mannheim) 1 h bei 37°C verdaut. Nach Auftrennen der entstandenen DNA-Fragmente auf einem 0,8 %igen niedrig-schmelzendem Agarosegel resultierte eine verschmierte Bahn von DNA-Bruchstücken, deren obere Begrenzung mit der Länge des Plasmids A59 übereinstimmte. Sämtliche DNAase I-Schnittstellen außerhalb der HindIII-Schnittstelle auf pBR327 führen zu einem Paar kleinerer Plasmidfragmente. Die Gelspur wurde mit einer Rasierklinge in kleine Segmente zerschnitten, so daß eine Reihe längenmäßig vorselektierter Fragmentfraktionen getrennt religiert werden konnten.

Die Ligationen wurden wie von Frischauf et al. beschrieben (XXV) in Anwesenheit der niedrigschmelzenden Agarose religiert und in E.coli HB101 transformiert (13 Ansätze). Es wurden durchschnittlich 50 Transformanten/Fraktion erhalten und jeweils 2 über ihre Plasmide analysiert. Die DNA-Sequenzen, die von den überlappenden Deletionsplasmiden erhalten wurden, ergaben die in Fig. 12 aufgeführte Gesamtsequenz für das Lu1220-D-Hydantoinase-Gen und die mitsequenzierten Randsequenzen.

Der kodierende Bereich des 3-Hydantoinasegens aus Lu 1220 erstreckt sich von Nukleotid 391 bis Nukleotid 1746. Die drei Nukleotide, die den Start der Translation signalisieren, und das Translationsstopsignal sind durch Schraffierung hervorgehoben.

2.6 Konstruktion von Lu 1220 D-Hydantoinase exprimierenden E.coli-Stämmen mit erhöhter Produktivität

Die für die Sequenzierung präparierten Fraktionen der A59-Delta 5'-Deletionsmutanten wurden als Ausgangsplasmide benutzt, um einen größeren Pool weiterer Deletionsmutanten zu erzeugen, deren Begrenzung dicht am ATG-Startkodon liegt. Dabei wurde von derjenigen Fraktion an Deletionsmutanten ausgegangen, deren HindIII-Linker in der Nähe der PvuII-Schnittstelle 52 Nukleotide oberhalb des Startkodons positioniert waren (Test auf Anwesenheit der Schnittstelle). Die Kolonien, die zu dieser Fraktion gehörten, wurden zusammengefaßt, und eine gemeinsame Plasmidpräparation wurde durchgeführt. Die DNA (10 µg) wurde mit HindIII linearisiert und kurz mit 2,4 U Bal31 behandelt in 100 µl (XXXIII) bei 25°C für 30 sec. Die Reaktion wurde durch Zugabe von Phenol gestoppt und die DNA aufgearbeitet. In einem Reaktionsansatz von 100 µl wurde die DNA mit DNA-Polymerase I 2 h bei 15°C (XXXIV) inkubiert, um die Enden der DNA bündig zu erhalten.

Es schloß sich dann eine Addition von BamH1-Linker (5'-CCGGATCCGG-3', P&L) an (1,5 µg DNA, ~1 pMol Enden, 84 pMol Linker, 20 U T4 DNA-Ligase, NEN) in 25 µl über Nacht bei 15°C. Dann folgte die Inaktivierung der Ligase bei 65°C für 10 min und Verdau der DNA mit BamH1. Die DNA wurde in 0,8 % niedrig-schmelzender Agarose als diffuse Bande gereinigt, isoliert und anschließend in 100 µl ligiert und in E.coli HB101 transfektiert. Insgesamt resultierten 1.822 Kolonien. 96 Kolonien wurden statistisch auf ihre DNA untersucht und über BamHI×PvuII-Restriktion kartiert. 14 Plasmide enthielten ein PvuII/BamHI-Fragment (~925 bp) und somit einen BamHI-Linker in direkter Nachbarschaft zum ATG-Startkodon und wurden vom BamHI-Linker aus ansequenziert. Es wurden nur die A + G und C + T-Reaktionen durchgeführt, da die exakte Sequenz der Region vorlag. Die Plasmide p70, p62 und p51 waren mit Bal31 bis an die in Fig. 12 beschriebenen Positionen verdaut worden. Der Übergang von den Vektorsequenzen zum Lu 1220 Genfragment ist jeweils mit einem Pfeil und dem Namen der entsprechenden Konstruktion in Fig. 12 eingezeichnet. Der Übergang erfolgt über einen BamH1-Linker an der Basis der Pfeile; die Linkersequenz ist in der Fig. 12 nicht eingezeichnet. Da eine Sequenzunsicherheit bezüglich der ersten 2 Nukleotide bestand, wurden bei Umbau dieser Plasmide in Expressionsvektoren alle drei Leserahmenmöglichkeiten berücksichtigt.

Als Expressionsvektoren standen zwei Systeme zur Verfügung: pEX31a-c und pCL547 (XXXVI). Die Vektoren pEX31a-c wurden von Prof. Dr. H. Schaller, Universität Heidelberg zur Verfügung gestellt, das Plasmid pCL547 von Dr. K. Stanley, EMBL, Heidelberg.

Die Plasmide pEX31a-c leiten sich von pBR322 ab, enthalten die pL-Promotorregion des Lambda-Phagen, die an die MS2-Polymerase Region gekoppelt ist (XXVI). Nach 97 Aminosäurekodons des Polymerasegens wurde die darauffolgende DNA-Sequenz über eine EcoRI-Schnittstelle in drei durchgängigen Leserahmen an eine Polylinkerregion angeknüpft. In die BamHI-Schnittstelle wurden die D-Hydantoinase-Gensequenzen der Plasmide p51, p62 und p70 ligiert. Dazu wurden die promotorhaltigen PstI-BamHI-Fragmente von pEX31a, b und c (Fig. 8) gegen das entsprechende Fragment von p51, p62 und p70 ausgetauscht. Die Fragmente wurden zunächst in 0,8 % niedrigschmelzender Agarose elektrophoretisch aufgetrennt, eluiert, gefällt und anschließend in Anwesenheit von 7 % Polyethylenglykol (XXXV) ligiert und in E.coli N4830 transfektiert.

Von den erhaltenen Klonen (pEX51a,b,c, pEX62a,b,c und pEX70a,b,c; ≈150 Klone/Ansatz) wurden jeweils 32 pro Leseraster in Mikrotiterplatten abgelegt. Die Kulturen (jeweils in 100 µl LB/Amp-Medium) wurden über Nacht bei 28°C geschüttelt. Bei dieser Temperatur sorgt der temperatursensible Lambda-Repressor CI857, der in E.coli N4830 chromosomal kodiert ist, für eine effektive Repression der Hydantoinase. Danach wurden zur Induktion der Enzymproduktion je 10 µl in eine frische Mikrotiterplatte übertragen und in 100 µl LB/Amp-Medium für 5 h bei 28°C geschüttelt. Im Wasserbad wurde dann die Kultur für 15 min auf 42°C erwärmt und weitere 3 h bei 42°C im Brutschrank geschüttelt. Anschließend wurden die E.coli-Bakterien mit je 20 µl 10 % SDS lysiert und wie in Beispiel 1 beschrieben auf Gegenwart von Lu1220-D-Hydantoinase-Antigen geprüft.

Das Ergebnis des immunologischen Testes auf Expression von Lu1220-D-Hydantoinase Antigen war im Fall der Plasmide pEX51 und pEX62 positiv im Leseraster b, negativ bei a und c; im Fall der Plasmide pEX70 waren alle Leseraster positiv. Jeweils 4 Repräsentanten der Antigen-positiven Mikrotiterkulturen pEX62b, pEX51b und pEX70a,b,c wurden in 100 ml Kulturen auf enzymatische Aktivitt der D-Hydantoinase untersucht. Als aktiv erwiesen sich nur pEX62b, pEX70a, pEX70b und pEX70c. Die Konstruktion pEX70 ist in Fig. 13 dargestellt.

Im Falle des Expressionsvektors pCL547 (XXXVI) stand nur ein Leseraster für eine D-Hydantoinase Genintegration zur Verfügung. Zwischen die singuläre BamH1-Schnittstelle zwischen cro und β-Galaktosidasegen und die PstI-Schnittstelle des pBR-Anteils (Fig. 14) wurden wie oben beschrieben die D-Hydantoinase-genhaltigen BamHI/PstI-Fragmente aus p70 und p62 und p51 integriert. Nach Transformation in E.coli N 4830 wurden je 4 Kolonien auf Expression enzymatisch aktiver D-Hydantoinase getestet. Da das D-Hydantoinasegen in diesen Konstruktionen unter der Kontrolle des Lambda-pr-Promotors/cI857-Repressors steht, wurde die Synthese von D-Hydantoinase nach Hitzeinduktion wie oben beschrieben gemessen. Als enzymatisch aktiv erwiesen sich nur die Konstruktionen pCL70. Sie ergab in Fermentationsansätzen (vgl. Beispiel 1.5) nach Hitzeinduktion eine 4-fach höhere Enzymaktivität, bezogen auf Biofeuchtmasse, als der Ausgangsstamm Lu1220.

Übersicht über die zitierte Literatur:


| | |
|---|---|
| I: | DE-OS 30 31 151 |
| II: | US-PS 4 237 224 |
| III: | EP-OS 41 313 |
| IV: | DE-OS 31 38 096 |
| V: | DE-OS 32 38 554 |
| VI: | J. Biochem. Tokyo 89, 667 (1981) |
| VII: | J. Mol. Biol. 77, 1 (1973) |
| VIII: | J. Biol. Chem. 242, 4409 (1968) |
| IX: | Gene 2, 95 (1977) |
| X: | Gene 9, 287 (1980) |
| XI: | Gene 11, 291 (1980) |
| XII: | Biotechnology Made Simple, A. Glossary of Recombinant DNA and Hybridoma Technology, PJB Publications, 18-20 Hill Rise, Richmond, Surrey, TW 106 UA, UK, 1983 |
| XIII: | Nature 215, 1285 (1967) |
| XIV: | Methods Enzym. 68, 281, 299 |

(1979)
XV: J. Mol. Biol. 41, 459 (1969)
XVI: Methods Enzym. 27, 942 (1973)
XVII: J. Biol. Chem. 243, 3557 (1968)
XVIII: Proc. Nat. Acad. Sci. 75, 2746 (1978)
XIX: The Bacteriophage Lambda, Hershey, A.D., ed. Cold Spring Harbor, New York 1971, Seiten 259, 628, 226 f, 565-588
XX: Molecular Cloning, A. Laboratory Manual, Cold Spring Harbor Laboratory, 1982, S. 86 ff
XXI: wie XX, S. 133
XXII: wie XX, S. 146, 245f, 286ff
XXIII: Gene 6, 23 (1979)
XXIV: Methods Enzym. 65, 499 (1980)
XXV: Nucl. Acids Res. 8, 5541 (1980)
XXVI: Gene 15, 81 (1981)
XXVII: Nucl. Acids Res. 11, 4077 (1983)
XXVIII: Affinity Chromatography, Principles and Methods, Pharmacia Fine Chemicals, June 1979, S. 12ff, 92ff)
XXIX: Nature 227, 680 (1970)
XXX: wie XX, S. 133, 297
XXXI: wie XX, S. 249ff
XXXII: wie XX, S. 113f, 394
XXXIII: wie XX, S. 135ff
XXXIV: wie XX, s. 108, 113f
XXXV: Nucl. Acids Res. 11, 7853 (1983)
XXXVI: EMBO J. 1, 1217 (1982)

## Patentansprüche

**1.** Verfahren zur Herstellung von mesophilen Mikroorganismen, die eine im thermophilen Temperaturbereich aktive D-Hydantoinase enthalten, dadurch gekennzeichnet, daß man
a) die DNA aus einem D-Hydantoin spaltenden thermophilen Mikroorganismus isoliert und in Bruchstücke zerlegt.
b) die so erhaltenen DNA-Bruchstücke mit einem Klonierungsvektor kombiniert,
c) die so erhaltenen rekombinanten Klonierungsvektoren in einen mesophilen Mikroorganismus integriert und
d) solche Mikroorganismen selektiert, die enzymatisch aktive Hydantoinase exprimieren.

**2.** D-Hydantoinase-kodierende DNA-Sequenz gemäß Fig. 6.

**3.** D-Hydantoinase-kodierende DNA-Sequenz gemäß Fig. 12.

**4.** Klonierungsvektor enthaltend eine D-Hydantoinase-kodierende DNA-Sequenz gemäß Anspruch 2 oder 3.

**5.** Durch Genmanipulation erhaltene mesophile Mikroorganismen, enthaltend einen Klonierungsvektor gemäß Anspruch 4.

**6.** Durch Genmanipulation erhaltene E.coli-Stäme, enthaltend einen Klonierungsvektor gemäß Anspruch 4.

**7.** Verwendung der Mikroorganismen gemäß Anspruch 5 zur Herstellung thermophiler D-Hydantoinasen.

**8.** Verwendung der E.coli-Stämme gemäß Anspruch 5 zur Herstellung thermophiler D-Hydantoinasen.

**9.** Verwendung von Mikroorganismen gemäß Anspruch 5 oder daraus hergestellten proteinextrakten zur stereoselektiven Spaltung von D,L-Hydantoinen in D-N-Carbamoyl-alpha-aminosäuren.

## Claims

**1.** A process for the preparation of mesophilic microorganisms which contain a D-hydantoinase which is active in the thermophilic temperature range, which process comprises
a) isolation and fragmentation of the DNA from a thermophilic microorganism which cleaves D-hydantoin,
b) combination of the resulting DNA fragments with a cloning vector,
c) integration of the resulting recombinant cloning vectors into a mesophilic microorganism and
d) selection of those microorganisms which express enzymatically active hydantoinase.

**2.** The DNA sequence coding for D-hydantoinase shown in Fig. 6.

**3.** The DNA sequence coding for D-hydantoinase shown in Fig. 12.

**4.** A cloning vector containing a DNA sequence coding for D-hydantoinase, as claimed in claim 2 or 3.

**5.** Mesophilic microorganisms obtained by gene manipulation and containing a cloning vector as claimed in claim 4.

**6.** E. coli strains obtained by gene manipulation and containing a cloning vector as claimed in claim 4.

7. Use of the microorganisms as claimed in claim 5 for the preparation of thermophilic D-hydantoinases.

8. Use of the E. coli strains as claimed in claim 5 for the preparation of thermophilic D-hydantoinases.

9. Use of the microorganisms as claimed in claim 5, or protein extracts prepared therefrom, for the stereoselective cleavage of D,L-hydantoins into D-N-carbamoyl-alpha-amino acids.

**Revendications**

1. Procédé pour la production de microorganismes mésophiles contenant une D-hydantoïnase active dans le domaine de température thermophile, caractérisé en ce que :
   a) on isole l'ADN à partir d'un microorganisme thermophile scindant la D-hydantoïne et on le décompose en fragments.
   b) on combine les fragments d'ADN ainsi obtenus avec un vecteur de clonation,
   c) on intègre les vecteurs de clonation recombinants ainsi obtenus dans un microorganisme mésophile, et
   d) on sélectionne des microorganismes qui expriment l'hydantoïnase possédant l'activité enzymatique.

2. La séquence d'ADN codant la D-hydantoïnase de la figure 6.

3. La séquence d'ADN codant la D-hydantoïnase de la figure 12.

4. Vecteur de clonation contenant une séquence d'ADN codant la D-hydrantoïnase selon la revendication 2 ou 3.

5. Microorganismes mésophiles obtenus par manipulation génétique, contenant un vecteur de clonation selon la revendication 4.

6. Souches d'E.Coli obtenues par manipulation génétique, contenant un vecteur de clonation selon la revendication 4.

7. Utilisation des microorganismes selon la revendication 5 pour la production de D-hydantoïnases thermophiles.

8. Utilisation des souches d'E.Coli selon la revendication 5 pour la préparation de D-hydantoïnases thermophiles.

9. Utilisation des microorganismes selon la revendication 5 ou d'extraits de protéines préparés à partir de ces microorganismes pour la scission stéréosélective de D,L-hydantoïnes en D-N-carbamoylalpha-aminoacides.

# FIG.1

Aminosäurehomologie

Aminosäurehomologie zwischen CBS 303.80- und Lu 1220-D-Hydantoinasen

```
              1                 10                  20                  30
CBS 303.80    P L L I K N G E I I T A D S R Y K A D I Y A E G E T I T R I G Q N L
Lu 1220       T K I I K N G T I V T A T D T Y E A D L L I K D G K I A R I G Q H L
```

53 % Homologie in den ersten 19 Aminosäuren

FIG.2

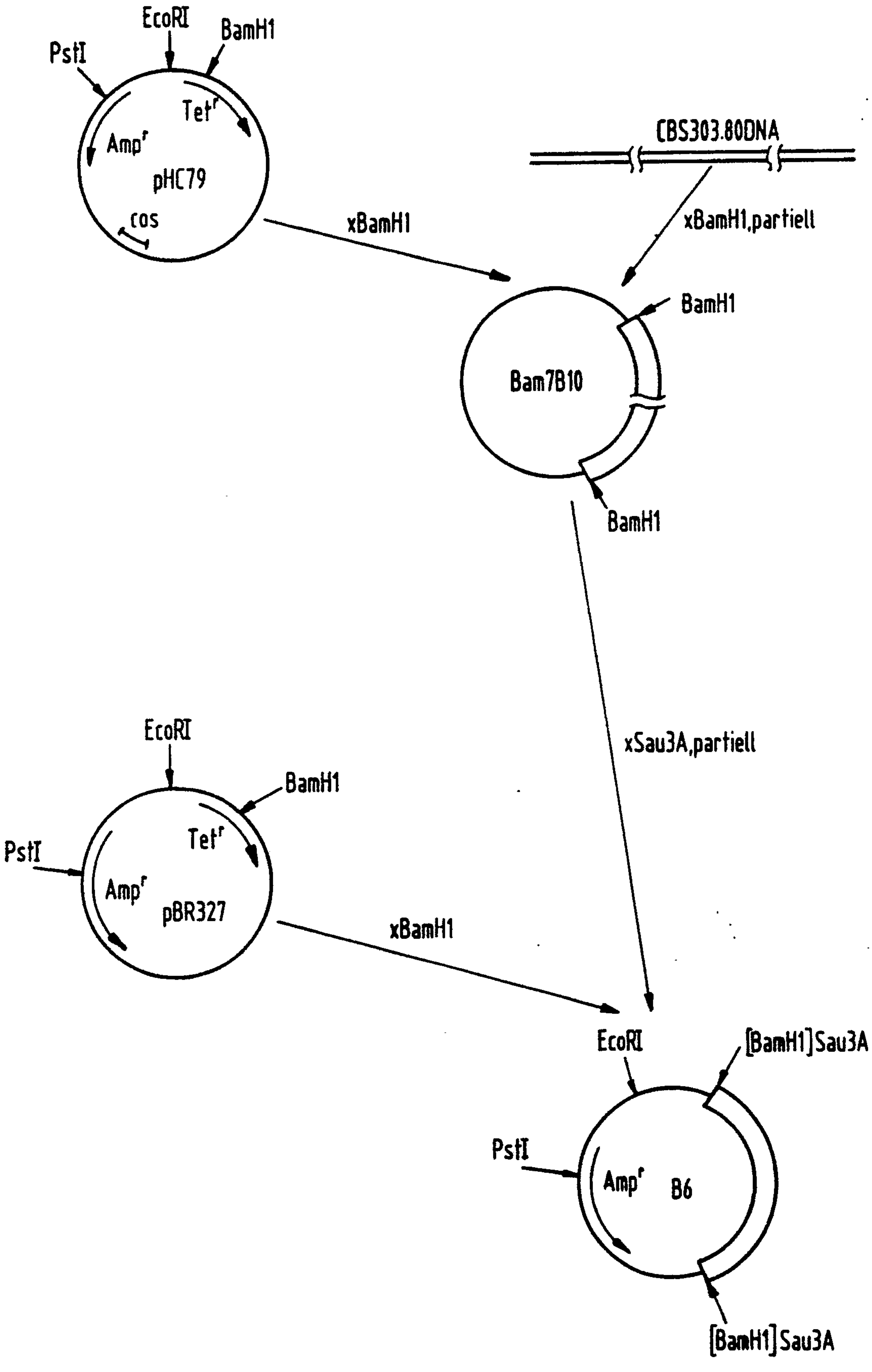
EcoRI
BamH1
PstI
Tet^r
Amp^r
pHC79
cos
xBamH1
CBS303.80DNA
xBamH1,partiell
BamH1
Bam7B10
BamH1
xSau3A,partiell
EcoRI
BamH1
PstI
Tet^r
Amp^r
pBR327
xBamH1
EcoRI
[BamH1]Sau3A
PstI
Amp^r
B6
[BamH1]Sau3A

FIG.3

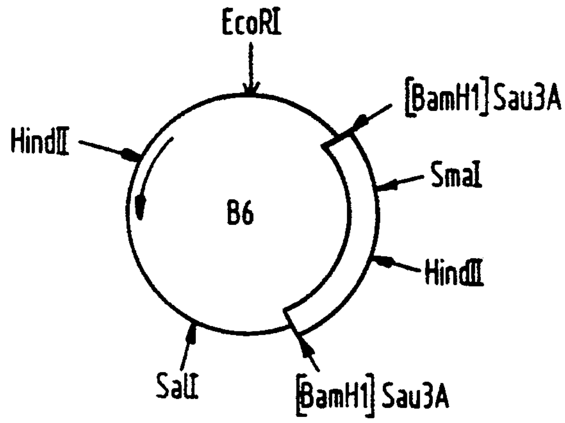
EcoRI
[BamH1] Sau3A
HindII
SmaI
B6
HindII
SalI
[BamH1] Sau3A

FIG.4

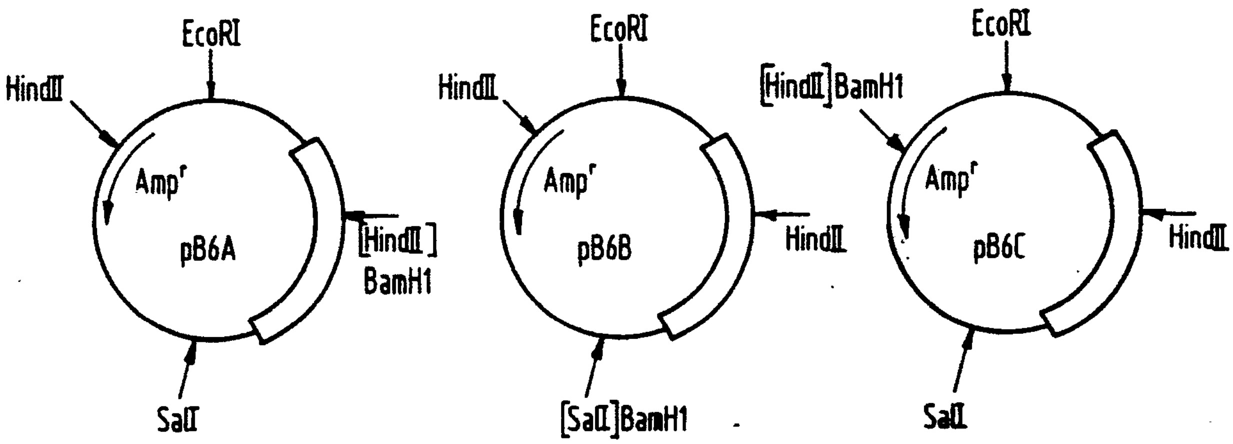
EcoRI
HindII
Amp^r
pB6A
[HindII]
BamH1
SalI
EcoRI
HindII
Amp^r
pB6B
HindII
[SalI]BamH1
EcoRI
[HindII]BamH1
Amp^r
pB6C
HindII
SalI

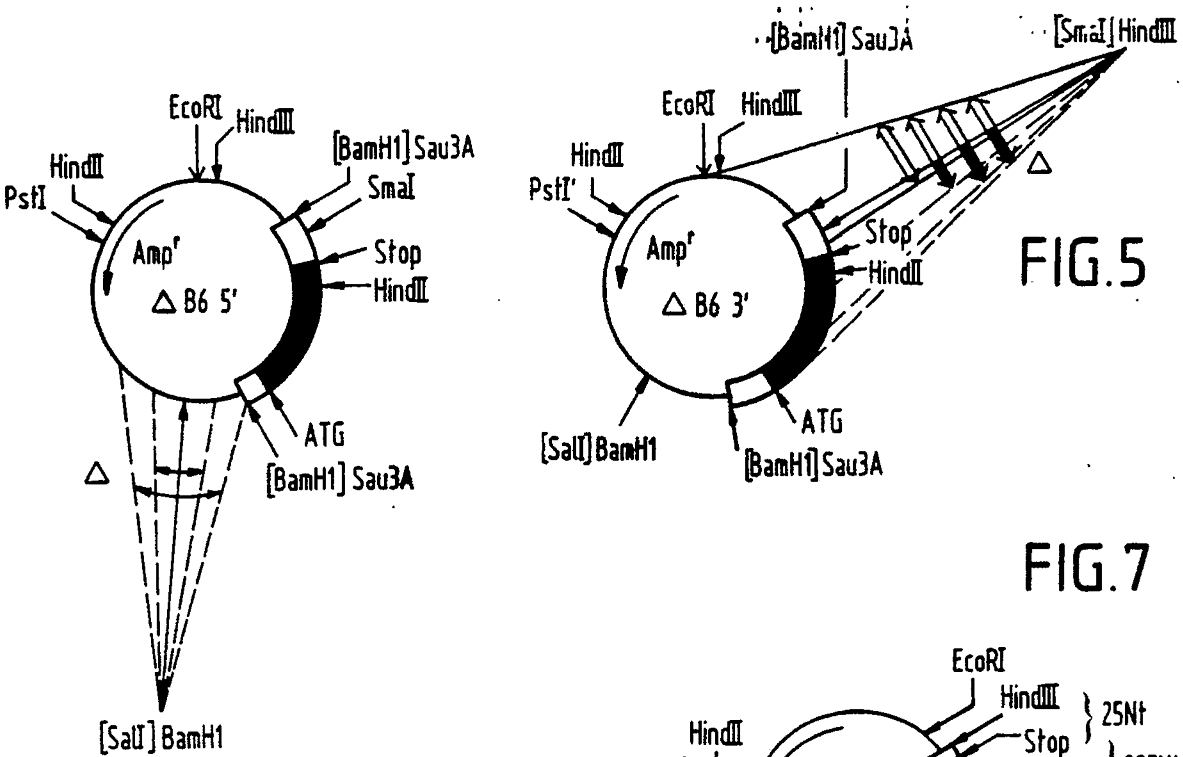

EcoRI
HindIII
HindII
PstI
[BamH1] Sau3A
SmaI
Amp^r
Δ B6 5'
Stop
HindII
ATG
[BamH1] Sau3A
Δ
[SalI] BamH1
[BamH1] Sau3A
[SmaI] HindIII
EcoRI HindIII
HindII
PstI'
Amp^r
Δ B6 3'
Stop
HindII
Δ
ATG
[SalI] BamH1
[BamH1] Sau3A


FIG.5

FIG.7

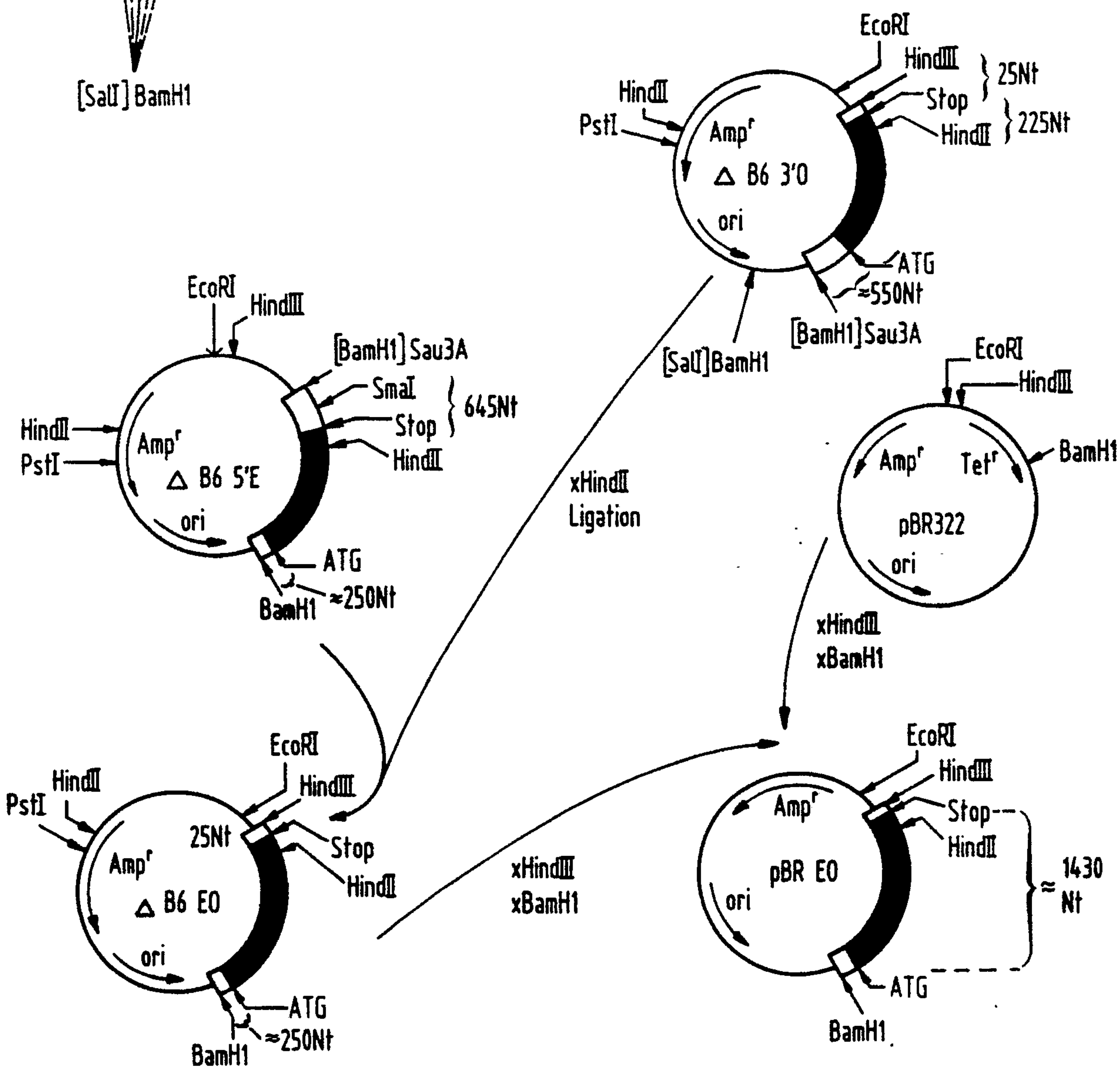

EcoRI
HindIII
25Nt
HindII
Stop
PstI
HindII
225Nt
Amp^r
Δ B6 3'O
ori
ATG
≈550Nt
[BamH1] Sau3A
[SalI] BamH1
EcoRI
HindIII
[BamH1] Sau3A
SmaI
645Nt
Stop
HindII
HindII
PstI
Amp^r
Δ B6 5'E
ori
ATG
BamH1
≈250Nt
xHindII
Ligation
EcoRI
HindIII
Amp^r
Tet^r
BamH1
pBR322
ori
xHindIII
xBamH1
EcoRI
HindIII
HindII
PstI
25Nt
Amp^r
Stop
HindII
Δ B6 EO
ori
ATG
BamH1
≈250Nt
xHindIII
xBamH1
EcoRI
HindIII
Amp^r
Stop
HindII
pBR EO
ori
≈ 1430 Nt
ATG
BamH1

# FIG.6a

```
  1- 50   CGTTGGAGAA AATATATGGC GCGTTTTCTT CAGGGGAAAC GGCTTTGCAA
 51-100   GTCCCNNAGC GTGCAATCGA CTTCGCTTTG TTGCGAAGGC CGAAGTTTGC
101-150   CTTGGGTCAG GCTATCGGTA TTTTATACTT GCAGAGTCTG TTAATGGAAG
                                     |-------> 3A8
151-200   AAACATTTCC ATCGGTACCA CCAACAGACC ATTTACACAA TAGCAAGACC
                          |---------> IF10
201-250   CAGTTCGGCA AATACGGTGG TTTGTTTTGT TGGACAACCA ACAGGATACG
          |--------> ID2
251-300   CTGTTGTGTA TGATGCCAGA TTCGTTGCTG ATTCCATAAG AAGAAAGTAC
301-350   GGCTTGTAAA CATAAGGGAG AAACGTATGC CTTTACTGAT CAAGAACGGC
351-400   GAAATCATCA CCGCGGACAG CCGGTACAAG GCCGACATCT ANGCNGAGGG
401-450   CGAGACCATC ACCCGCATCG GGCAGAACCT CGAGGCCCCG CCCGGCACCG
451-500   AGGTGATAGC CCACCGGCAA GTACGTGTTT CCCGGCTTCA TAGACCCCCA
501-550   CGTGCACATC TACCTGCCCT TCATGGCCAC CTTCGCCAAG GACACCCACG
551-600   AGACCGGCTC CAAGGCGGCC TTGATGGGGG GCACCACCAC CTACATCGAG
601-650   ATGTGCTGCC CCAGCCGCAA CGACGAGCCC TCGAGGGCTA CCAGCTCTGG
651-700   AAGAGCAAGC CGAGGGCAAC AGCTACTGCG ATTACACCTT CCACATGGCC
701-750   GTCCCCAAGT TCGACGAAAA AACCGAGGGG CAGCTGCGGG AGATTGTGCC
751-800   GACGGCATTA GCTCCTTCAA AATTTTTCTC TCCTACAAAA ACTTCTTTGG
801-850   CGTGGACGAC GGGGAGATGT ACCAGACCCT GCGCCTAGCC AAGTGAGCTG
```

FIG.6b

| | |
|---|---|
| 851-900 | GGGGTGATCG TGACCGCCCA CTGTGAGAAN NNNNNNCGAT AAAACCTACG |
| 901-950 | CCGAGCGGGG CGGGGTGGAG GCCATGAAGT ACATCATGTC GCCGNNCGAT |
| 951-000 | AAGCGCAACC AGAAAGTCCT GTGGGGCCCT GCCCAGGGCT TCATCGACAC |
| 1001-050 | CGTGGGCACC GACCACTGCC CCTTCGACAC CGGCAGAAGC TGCTGGGCAA |
| 1051-100 | GGAGGCCTTC ACCGCTATTC CCAACGGCAC CCGGCCATCG AAGACCGGGT |
| 1101-150 | CAACCTGCTC TACACCTACG GGGTGGACCG CGGCCGCCTC GATATTCACC |
| 1151-200 | GCTTTGTGGN GGCTCAGCAC CAAGGCCGCC AAGTTGTTTG GACTGTTCCC |
| 1201-250 | CCGCNNNNNN NNNAACAACG ACTACAACGG CTTCGAGGGC TTTGAGATTG |
| 1251-300 | ACGGCCGGCC CAGCGTGGTG ACGGTGCGGG GTAAGGTGGC GGTGCGGGAC |
| 1301-350 | GGGCAGTTTG TGGGCGAGAA GGGGTGGGGT AAGCTCCTGC GGCGCGAGCA |
| 1351-400 | TGTACTTCTA AATGAAGCCG AGATGGGTTT TGNATTTGCT GGGTGGGATC |
| 1401-450 | TGCCTGTGGA TAGCGGTGGT GCTGGTGCTG GGGGGTAGTT CGGCCTGGGC |
| 1451-500 | GCTTTTTGCG GTAGTCGGCG AACTCCTGTT GGTGCTGGCC CAAAGAGGCT |
| 1501-550 | TCAAGCGGAG ATAGATGGTA GGTGAATAAT CCTGACGGGC AGCCGCCATA |
| 1551-600 | AATAGGGAAG ACCATGATGC AAGCCAATAC CTCTCCAGAG CTGTCCGCCA |
| 1601-650 | AAGGTTCGAA CGCAGCCGCC GTTTCGGTTC AGGGTGTTTC GATGGTGTTT |
| 1651-700 | CCCAACGGAA CCGTGGCCCT CAAAGACGCC AACCTCGAGA TCGCCNAGGG |
| 1701-701 | G |

FIG.8

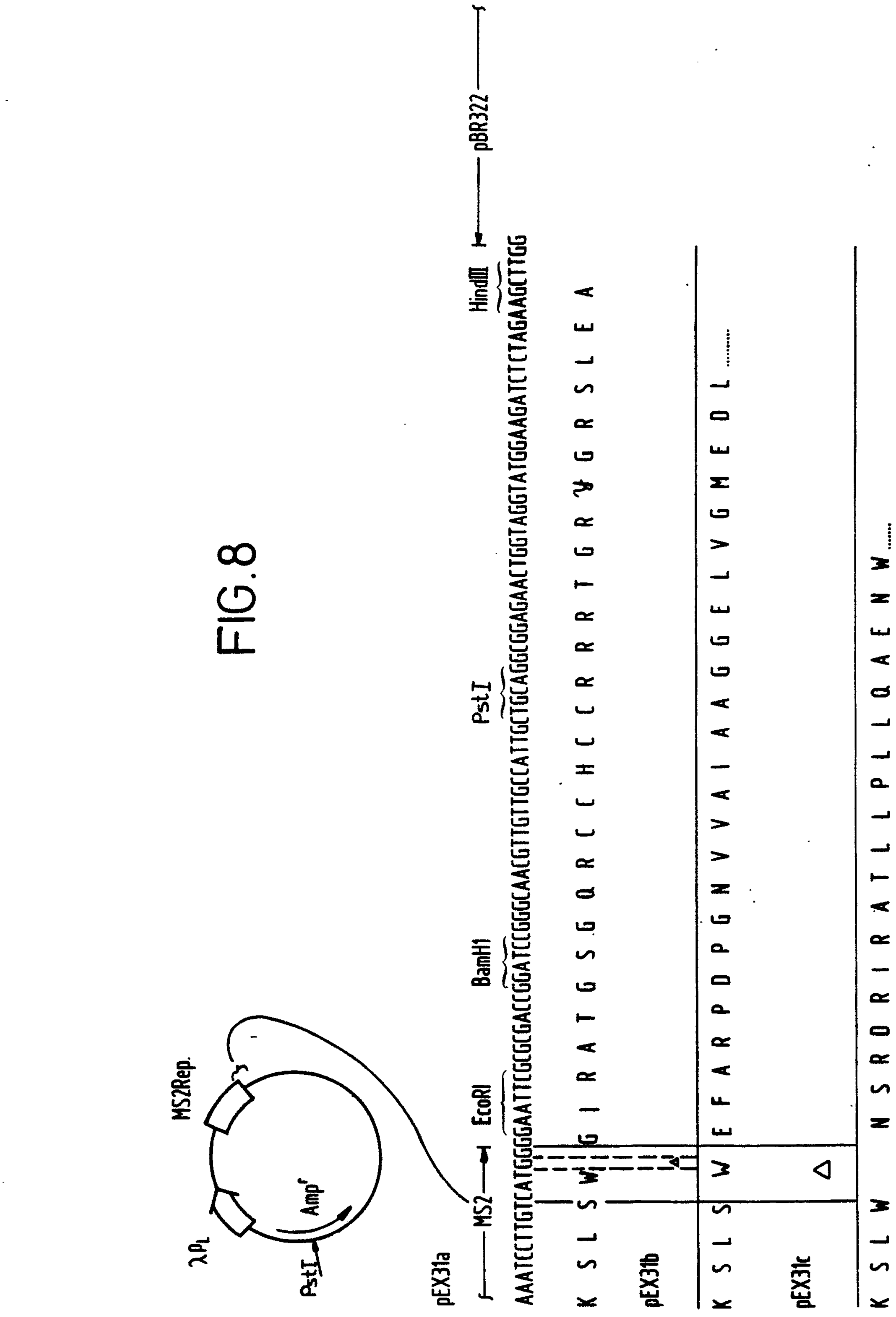
λPL
PstI
Ampr
MS2Rep.
pEX31a
MS2
EcoRI
BamH1
PstI
HindIII
pBR322
AAATCTTGTCATGGGGAATTCGCGGACCGGATCCGGGCAACGTTGTTGCCATTGCTGCAGGCGGAGAACTGGTAGGTATGGAAGATCTCTAGAAGCTTGG
K S L S W G I R A T G S G Q R C C H C C R R R T G R * G R S L E A
pEX31b
K S L S W E F A R P D P G N V V A I A A G G E L V G M E D L ........
pEX31c
Δ
K S L W N S R D R I R A T L L P L L Q A E N W.......

FIG.9

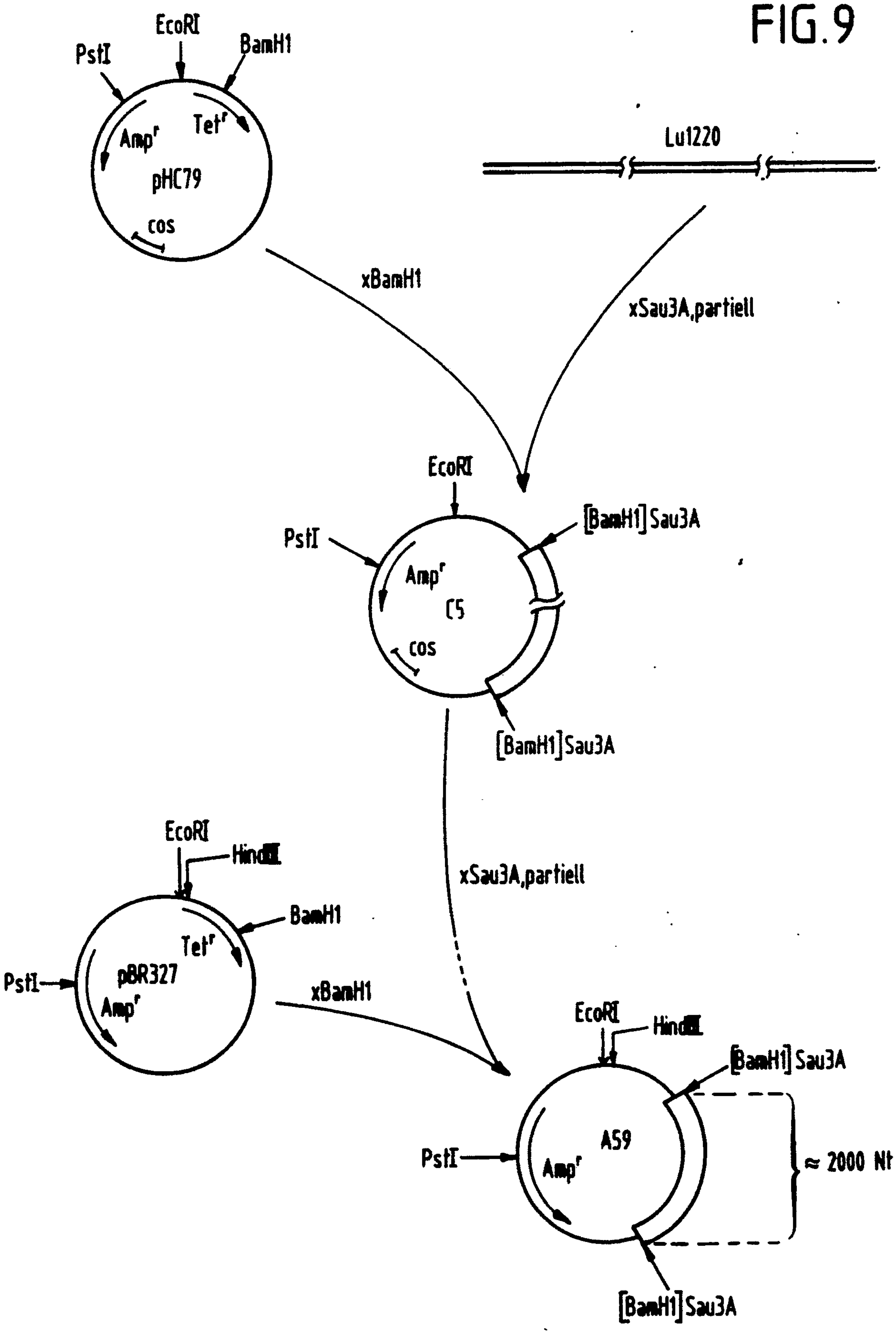
PstI
EcoRI
BamH1
Amp^r
Tet^r
pHC79
cos
Lu1220
xBamH1
xSau3A,partiell
EcoRI
PstI
[BamH1] Sau3A
Amp^r
C5
cos
[BamH1] Sau3A
xSau3A,partiell
EcoRI
HindIII
BamH1
Tet^r
PstI
pBR327
Amp^r
xBamH1
EcoRI
HindIII
[BamH1] Sau3A
A59
PstI
Amp^r
≈ 2000 Nt
[BamH1] Sau3A

FIG.10

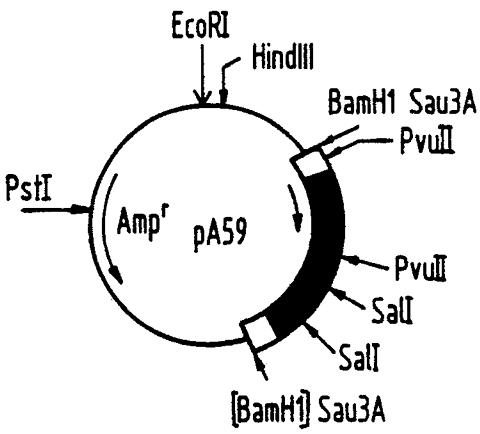
EcoRI
HindIII
BamH1 Sau3A
PvuII
PstI
Amp^r
pA59
PvuII
SalI
SalI
[BamH1] Sau3A

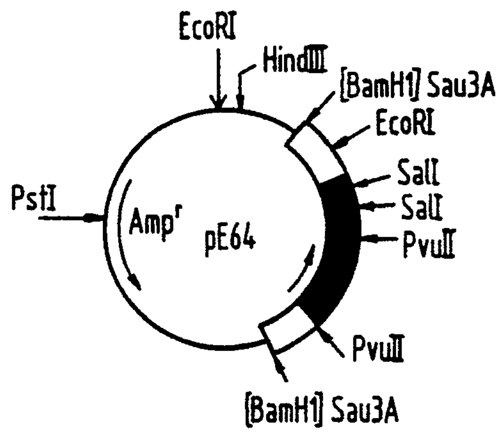
EcoRI
HindIII
[BamH1] Sau3A
EcoRI
SalI
SalI
PvuII
PstI
Amp^r
pE64
PvuII
[BamH1] Sau3A

# FIG.12a

1-050 CGGTTATGGA TATTGTTGGG AAGTCAGTTC ATAAATATTC CAATTGGGGG

51-100 GACCTCGACC TTCATTATAA AGTGGTTGTT CGCATTAACA AAGATGTTTG

101-150 CATTAATTGC AATAAATGTT ATATCTCCTG TGAAGATGCT TCTCATCAAT

151-200 GCATTGATCG TTTAACGGAT GAAAATGGAA AAGAGTATTT AAAAGTGCGC

201-250 GAAGAAGATT GCGTAGGGTG TAATTTATGT TCGATCGTCT GTCCGGTGGA

251-300 TGGTGCGATT GACATGGTCG AAATGCCAAG CGACAATCTG CCGATGACAT

301-350 GGAATGAACG CCAAGCGGCC ATTAGCGGGC TGAGCAGCTG TAGCGTTGAT

p70 p62

351-400 GTGAAATAAA ACGAAATTTC CAGCGGAGGA GGATGTTGAA ATGACAAAAA

p51

401-450 TAATAAAAAA TGGAACGATT GTTACCGCAA CCGATACGTA TGAAGCGGAC

451-500 TTGCTCATTA AAGACGGAAA AATTGCCATG ATAGGCCAAC ATTTAGAAGA

501-550 AAAAGGCGCT GAAGTGATTG ATGCCAAAGG CTGTTACGTA TTTCCAGGCG

551-600 GTATTGATTC GCACACGCAT TTAGATATGC CGTTTGGCGG CACGGTGACA

601-650 AAGGATGATT TCGAATCTGG AACGATTGCG GCGGCATTTG GCGGAACAAC

651-700 GACCATCATC GACTTTTGTT TAACGAATAA AGGGGAGCCA TTAAAAAAAG

701-750 CGATTGAAAC TTGGCACAAC AAAGCGAAGG GAAAAGCGGT TATTGATTAT

751-800 GGCTTCCATT TAATGATTAG CGAAATTACG GATGACGTAT TAGAAGAGCT

801-850 GCCAAAAGTC ATTGCCGAAG AAGGGATAAC ATCCTTTAAA GTGTTTATGG

851-900 CGTATAAAAA CGTATTTCAG GCAGATGATG GAACGTTATA CCGCACGCTA

901-950 GTGGCTGCCA AAGAACTTGG CGCGCTTGTC ATGGTTCATG CGGAAAATGG

FIG.12b

| | |
|---|---|
| 951-000 | GGATGTGATT GATTACTTAA CGAAAAAAGC GCTTGCGGAA GGGAATACGG |
| 1001-050 | AGCCGATTTA CCATGCTTTA ACGCGGCCTC CAGAAGTAGA AGGAGAAGCG |
| 1051-100 | ACCGGGCGCG CCTGTCAATT GACAGAGCTT GCCGGTTCAC AACTTTACGT |
| 1101-150 | TGTTCACGTG ACATGTGCGC AAGCGGTGGA AAAAATTGCA CAAGCGCGCA |
| 1151-200 | ATAAAGGGTT GGATGTGTGG GGAGAAACGT GTCCGCAATA TCTTGTTCTC |
| 1201-250 | GACCAATCGT ATTTAGAAAA GCCTGATTTT GAAGGCGCGA AATATGTTTG |
| 1251-300 | GTCCCCTCCG CTTCGTGAAA AATGGCATCA AGAAGTATTG TGGAATGCGC |
| 1301-350 | TGAAAAACGG CCAGCTGCAA ACGCTTGGAT CGGACCAATG TTCATTTGAC |
| 1351-400 | TTTAAAGGCC AAAAAGAACT TGGCAGAGGA GATTTTACTA AAATTCCAAA |
| 1401-450 | CGGCGGGCCG ATGGTCGAGG ATCGGGTCAG CATTCTTTTC AGTGAAGGGG |
| 1451-500 | TTAAAAAAGG AAGAATCACG TTAAATCAAT TTGTCGATAT TATGTCGACA |
| 1501-550 | AGAATTGCCA AATTGTTCGG GTTATTCCCG AGAAAAGGAA CGATCGCGGT |
| 1551-600 | AGGTTCAGAC GCAGACTTAG TCATTTTTGA CCCGGATATC GAACGGGTGA |
| 1601-650 | TTTCGGCGGA AACACACCAT ATGGCCGTCG ACTATAATGC ATTTGAAGGA |
| 1651-700 | ATGAAAGTAA CGGGTGAACC GGTATCGGTT CGTGCAGAGG CGAATTTGTT |
| 1701-750 | GTCCGTGATA AACAATTTGT CGGAAAACCA GGGTACGGCC AATATTTAAA |
| 1751-800 | ACGGGCAAAA TACGGAACCT CAAAGATTTC CAAGCAGAAC GAGAAATTAA |
| 1801-850 | CCATTTAAAA GAATAACAAC CTACTCTTGC CCCTTAAAAT GCCAATAAAA |
| 1851-900 | TGCAACACTT AGCTTTATTC CCGTTCTAAG CAG |

FIG.13

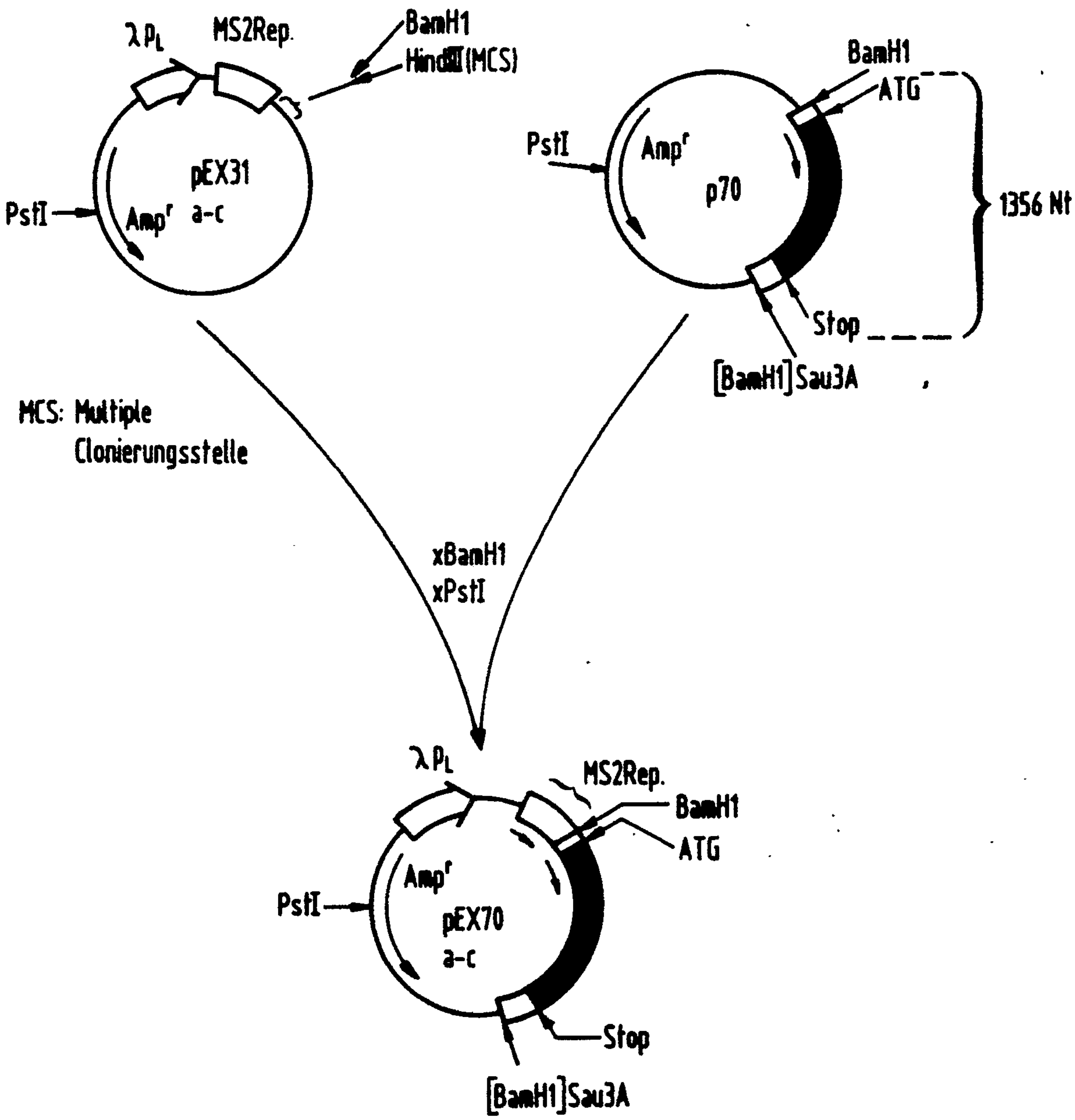
λ$p_L$
MS2Rep.
BamH1
HindIII(MCS)
PstI
Amp^r
pEX31
a-c
BamH1
ATG
PstI
Amp^r
p70
1356 Nt
Stop
[BamH1]Sau3A
MCS: Multiple
Clonierungsstelle
xBamH1
xPstI
λ$p_L$
MS2Rep.
BamH1
ATG
Amp^r
PstI
pEX70
a-c
Stop
[BamH1]Sau3A

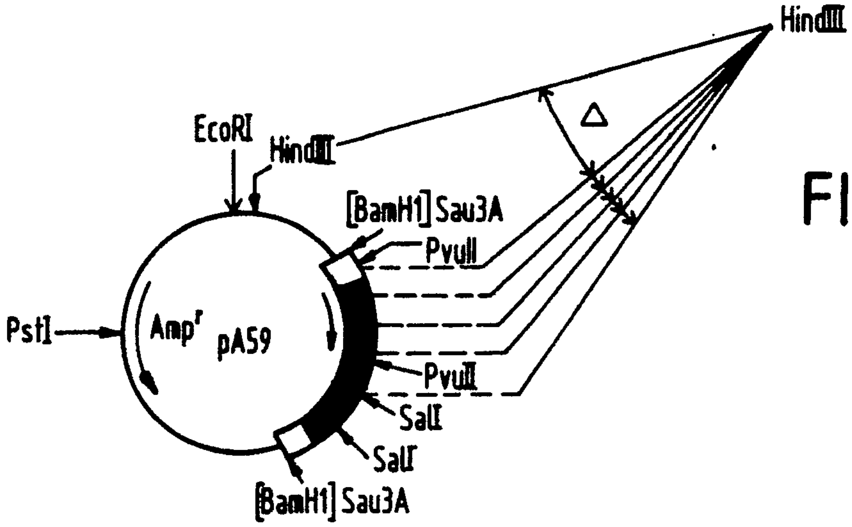
HindIII
EcoRI
HindIII
Δ
[BamH1] Sau3A
PvuII
PstI
Amp^r
pA59
PvuII
SalI
SalI
[BamH1] Sau3A

FIG.11

FIG.14

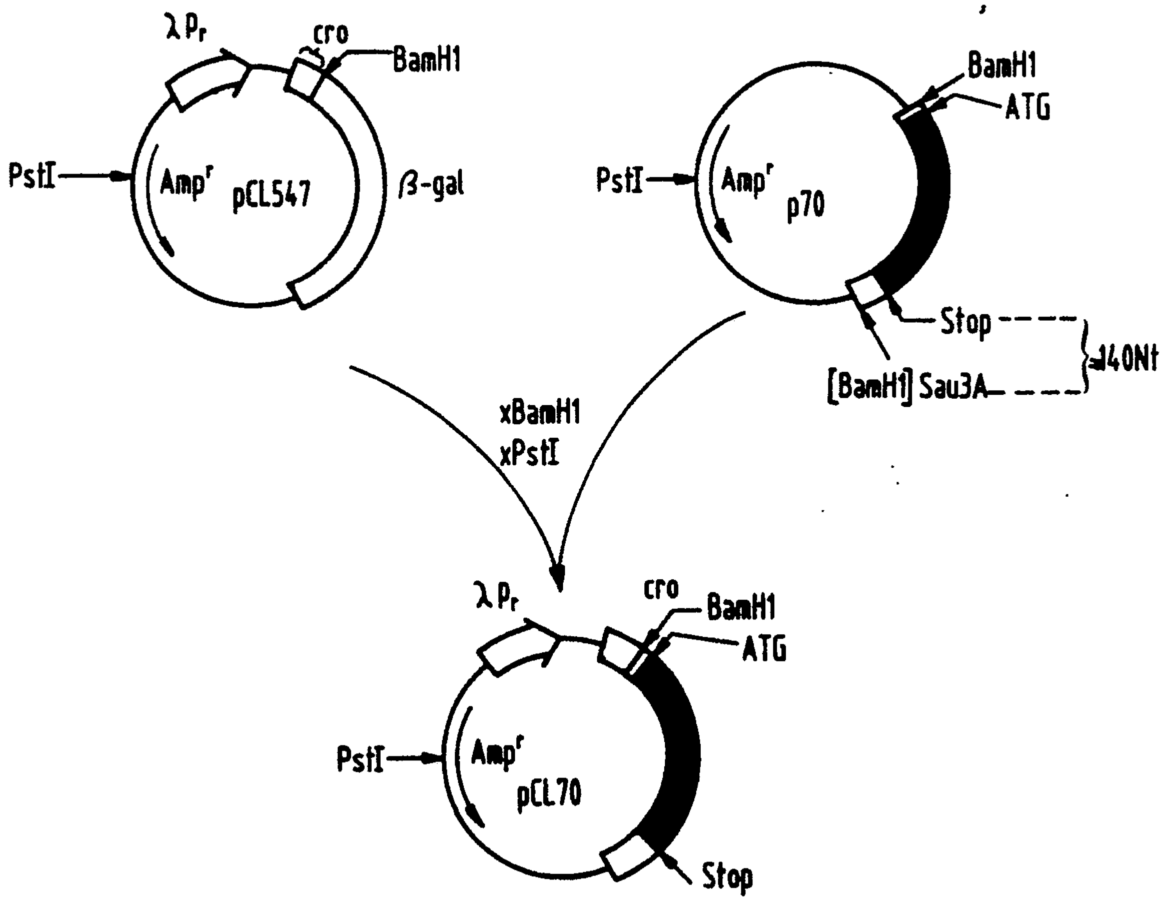
λPr
cro
BamH1
PstI
Ampr
pCL547
β-gal
BamH1
ATG
PstI
Ampr
p70
Stop
≃140Nt
[BamH1] Sau3A
xBamH1
xPstI
λPr
cro
BamH1
ATG
PstI
Ampr
pCL70
Stop